# EUROPEAN PATENT APPLICATION

(11) **EP 2 684 870 A1**
(43) Date of publication of application: **15.01.2014**
(21) Application number: 12745032.8
(22) Date of filing: 06.02.2012
(51) Int. Cl.: C07C 327/22, C07C 327/26

(54) **METHOD FOR PREPARING THIOCARBOXYLIC ACID S-(FLUOROALKYL) ESTER**

(30) Priority: 09.02.2011 JP 2011025916
(71) Applicant: Sumitomo Chemical Company Limited, Tokyo 104-8260 (JP)
(72) Inventor: TABUCHI, Takanori, Oita-shi, Oita 870-0106 (JP)
(74) Representative: Vossius & Partner
(86) International application number: PCT/JP2012/053199
(87) International publication number: WO 2012/108542

(57) **Abstract**

It is provided that a method for producing a thiocarboxylic acid S-(fluoroalkyl) ester represented by formula (III) [wherein R¹ represents a methyl group, an ethyl group or a phenyl group, and R² represents a C1-C5 fluoroalkyl group] by reacting a thiocarboxylic acid represented by formula (I) [wherein R¹ has the same meaning as defined above] with a fluoroolefin represented by formula (II) [wherein R² has the same meaning as defined above] in the presence of a radical generator.

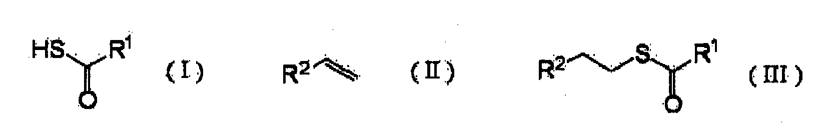

## Description

### TECHNICAL FIELD

The present invention relates to a method for producing a thiocarboxylic acid S-(fluoroalkyl) ester.

### BACKGROUND ART

Patent Document 1 and Patent Document 2 describe that a thiocarboxylic acid S-(fluoroalkyl) ester such as S-(3,3,3-trifluoropropyl) thioacetate and S-(3,3,3-trifluoropropyl) thiobenzoate is a synthetic intermediate for an organic sulfur compound that gives an excellent control effect against harmful arthropods. In addition, Patent Document 3, Non-Patent Document 1 and Non-Patent Document 2 describe that a thiocarboxylic acid S-(fluoroalkyl) ester such as S-(4,4,5,5,5-pentafluoropentyl) thioacetate is a synthetic intermediate for pharmaceuticals such as a therapeutic agent for cancer.

For producing the thiocarboxylic acid S-(fluoroalkyl) esters described above, a method for reacting a thiocarboxylic acid with a fluoroalkyl halide in the presence of a base, as well as an ionic reaction for reacting a thiocarboxylic acid with a fluoroalkyl alcohol in the presence of diethyl azodicarboxylate and triphenylphosphine, that is a Mitsunobu reaction, are known.

### PRIOR ART DOCUMENTS

### PATENT DOCUMENTS

Patent Document 1: US 2010/0160430A1
Patent Document 2: JP-A-2009-256302
Patent Document 3: WO 2005/077968

### NON-PATENT DOCUMENTS

Non-Patent Document 1: Tetrahedron Letters, vol. 35, page 9141, (1994)
Non-Patent Document 2: Bioorganic & Medicinal Chemistry, vol. 14, page 4803, (2006)

### SUMMARY OF THE INVENTION

### PROBLEMS TO BE SOLVED BY THE INVENTION

The present invention provides a novel method for producing a thiocarboxylic acid S-(fluoroalkyl) ester.

### MEANS FOR SOLVING THE PROBLEMS

The present inventors have studied a novel method for producing a thiocarboxylic acid S-(fluoroalkyl) ester, and consequently have reached the following invention. Namely, the present invention is as described below.
[1] A method for producing a thiocarboxylic acid S-(fluoroalkyl) ester represented by formula (III) 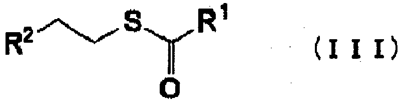 (wherein R¹ represents a methyl group, an ethyl group or a phenyl group, and R² represents a C1-C5 fluoroalkyl group) which comprises reacting a thiocarboxylic acid represented by formula (I) 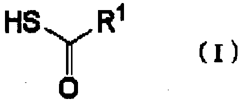 (wherein R¹ has the same meaning as defined above) with a fluoroolefin represented by formula (II) 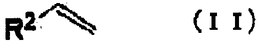 (wherein R² has the same meaning as defined above) in the presence of a radical generator.
[2] The production method according to [1], wherein the radical generator is an azo compound represented by formula (IV)

   **R³-N=N-R⁴** (IV)

   (wherein R³ and R⁴ are the same or di fferent, and represent a C2-C12 alkyl group that is optionally substituted or a carbamoyl group).
[3] The production method according to [1], wherein the radical generator is 2,2'-azobis(4-methoxy-2,4-dimethylvaleronitrile).
[4] The production method according to any of [1] to [3], wherein R¹ is a methyl group.
[5] The production method according to any of [1] to [4], wherein R² is a C1-C5 perfluoroalkyl group.
[6] The production method according to any of [1] to [5], wherein R² is a trifluoromethyl group.
[7] The production method according to any of [1] to [6], comprising reacting under atmospheric pressure using an organic solvent.

### EFFECT OF THE INVENTION

According to the present invention, a novel method for producing a thiocarboxylic acid S-(fluoroalkyl) ester can be provided.

### MODE FOR CARRYING OUT THE INVENTION

First, a thiocarboxylic acid represented by formula (I) 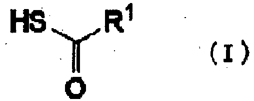 (hereinafter described as thiocarboxylic acid (I)) will be explained.

R¹ represents a methyl group, an ethyl group or a phenyl group, and among them, a methyl group is preferable.

Namely, the thiocarboxylic acid (I) is thioacetic acid, thiopropionic acid, or thiobenzoic acid, and among them, thioacetic acid is preferable.

Next, a fluoroolefin represented by formula (II) 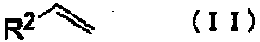 (hereinafter referred to as olefin (II)) will be explained.

R² represents a C1-C5 fluoroalkyl group.

Examples of the C1-C5 fluoroalkyl group include C1 fluoroalkyl groups such as a fluoromethyl group, a difluoromethyl group and a trifluoromethyl group;

C2 fluoroalkyl groups such as a 1-fluoroethyl group, a 2-fluoroethyl group, a 1,1-difluoroethyl group, a 1,2-difluoroethyl group, a 2,2-difluoroethyl group, a 1,1,2-trifluoroethyl group, a 1,2,2-trifluoroethyl group, a 2,2,2-trifluoroethyl group, a 1,1,2,2-tetrafluoroethyl group, a 1,2,2,2-tetrafluoroethyl group and a pentafluoroethyl group;

C3 fluoroalkyl groups such as a 1-fluoropropyl group, a 2-fluoropropyl group, a 3-fluoropropyl group, a 1,1-difluoropropyl group, a 1,2-difluoropropyl group, a 1,3-difluoropropyl group, a 2,2-difluoropropyl group, a 2,3-difluoropropyl group, a 3,3-difluoropropyl group, a 1,1,2-trifluoropropyl group, a 1,1,3-trifluoropropyl group, a 1,2,2-trifluoropropyl group, a 1,2,3-trifluoropropyl group, a 1,3,3-trifluoropropyl group, a 2,3,3-trifluoropropyl group, a 3,3,3-trifluoropropyl group, a 1,1,2,2-tetrafluoropropyl group, a 1,1,2,3-tetrafluoropropyl group, a 1,2,2,3-tetrafluoropropyl group, a 1,2,3,3-tetrafluoropropyl group, a 1,3,3,3-tetrafluoropropyl group, a 2,2,3,3-tetrafluoropropyl group, a 2,3,3,3-tetrafluoropropyl group, a 1,1,2,2,3-pentafluoropropyl group, a 1,2,3,3,3-pentafluoropropyl group, a 2,2,3,3,3-pentafluoropropyl group, a 1,2,2,3,3,3-hexafluoropropyl group, a heptafluoropropyl group and a 2,2,2-trifluoro-(1-trifluoromethyl)ethyl group;

C4 fluoroalkyl groups such as a 1-fluorobutyl group, a 2-fluorobutyl group, a 3-fluorobutyl group, a 4-fluorobutyl group, a 1,1-difluorobutyl group, a 1,2-difluorobutyl group, a 1,3-difluorobutyl group, a 1,4-difluorobutyl group, a 2,2-difluorobutyl group, a 2,3-difluorobutyl group, a 2,4-difluorophenyl group, a 3,3-difluorobutyl group, a 3,4-difluorobutyl group, a 4,4-difluorobutyl group, a 1,1,2-trifluorobutyl group, a 1,1,3-trifluorobutyl group, a 1,1,4-trifluorobutyl group, a 1,2,2-trifluorobutyl group, a 1,2,3-trifluorobutyl group, a 1,2,4-trifluorobutyl group, a 1,3,3-trifluorobutyl group, a 1,3,4-trifluorobutyl group, a 1,4,4-trifluorobutyl group, a 2,2,3-trifluorobutyl group, a 2,2,4-trifluorobutyl group, a 2,3,3-trifluorobutyl group, a 2,3,4-trifluorobutyl group, a 2,4,4-trifluorobutyl group, a 3,3,4-trifluorobutyl group, a 3,4,4-trifluorobutyl group, a 4,4,4-trifluorobutyl group, a 1,1,2,2-tetrafluorobutyl group, a 1,1,2,3-tetrafluorobutyl group, a 1,1,2,4-tetrafluorobutyl group, a 1,1,3,3-tetrafluorobutyl group, a 1,1,3,4-tetrafluorobutyl group, a 1,1,4,4-tetrafluorobutyl group, a 1,2,2,3-tetrafluorobutyl group, a 1,2,2,4-tetrafluorobutyl group, a 1,2,3,4-tetrafluorobutyl group, a 1,2,4,4-tetrafluorobutyl group, a 1,3,3,4-tetrafluorobutyl group, a 1,3,4,4-tetrafluorobutyl group, a 1,4,4,4-tetrafluorobutyl group, a 2,2,3,3-tetrafluorobutyl group, a 2,2,3,4-tetrafluorobutyl group, a 2,2,4,4-tetrafluorobutyl group, a 2,3,3,4-tetrafluorobutyl group, a 2,3,4,4-tetrafluorobutyl group, a 2,4,4,4-tetrafluorobutyl group, a 3,3,4,4-tetrafluorobutyl group, a 3;4,4,4-tetrafluorobutyl group, a 1,1,2,2,3-pentafluorobutyl group, a 1,1,2,2,4-pentafluorobutyl group, a 1,1,2,3,3-pentafluorobutyl group, a 1,1,2,3,4-pentafluorobutyl group, a 1,1,2,4,4-pentafluorobutyl group, a 1,1,3,3,4-pentafluorobutyl group, a 1,1,3,4,4-pentafluorobutyl group, a 1,1,4,4,4-pentafluorobutyl group, a 1,2,2,3,3-pentafluorobutyl group, a 1,2,2,3,4-pentafluorobutyl group, a 1,2,2,4,4-pentafluorobutyl group, a 1,2,3,3,4-pentafluorobutyl group, a 1,2,3,4,4-pentafluorobutyl group, a 1,2,4,4,4-pentafluorobutyl group, a 1,3,3,4,4-pentafluorobutyl group, a 1,3,4,4,4-pentafluorobutyl group, a 2,2,3,3,4-pentafluorobutyl group, a 2,2,3,4,4-pentafluorobutyl group, a 2,2,4,4,4-pentafluorobutyl group, a 2,3,3,4,4-pentafluorobutyl group, a 2,3,4,4,4-pentafluorobutyl group, a 3,3,4,4,4-pentafluorobutyl group, a 1,1,2,2,3,3-hexafluorobutyl group, a 1,1,2,2,3,4-hexafluorobutyl group, a 1,1,2,2,4,4-hexafluorobutyl group, a 1,1,2,3,3,4-hexafluorobutyl group, a 1,1,2,3,4,4-hexafluorobutyl group, a 1,1,2,4,4,4-hexafluorobutyl group, a 1,1,3,3,4,4-hexafluorobutyl group, a 1,1,3,4,4,4-hexafluorobutyl group, a 1,2,2,3,3,4-hexafluorobutyl group, a 1,2,2,3,4,4-hexafluorobutyl group, a 1,2,2,4,4,4-hexafluorobutyl group, a 1,2,3,3,4,4-hexafluorobutyl group, a 1,2,3,4,4,4-hexafluorobutyl group, a 1,3,3,4,4,4-hexafluorobutyl group, a 2,2,3,3,4,4-hexafluorobutyl group, a 2,3,3,4,4,4-hexafluorobutyl group, a 1,1,2,2,3,3,4-heptafluorobutyl group, a 1,1,2,2,3,4,4-heptafluorobutyl group, a 1,1,2,2,4,4,4-heptafluorobutyl group, a 1,2,2,3,3,4,4-heptafluorobutyl group, a 1,2,2,3,4,4,4-heptafluorobutyl group, a 1,2,3,3,4,4,4-heptafluorobutyl group, a 2,2,3,3,4,4,4-heptafluorobutyl group, a 1,1,2,2,3,3,4,4-octafluorobutyl group, a 1,2,2,3,3,4,4, 4-octafluorobutyl group, a 1,1,2,3,3,4,4,4-octafluorobutyl group, a 1,1,2,2,3,4,4,4-octafluorobutyl group and a nonafluorobutyl group; and

C5 fluoroalkyl group such as a 1-fluoropentyl group, a 2-fluoropentyl group, a 3-fluoropentyl group, a 4-fluoropentyl group, a 5-fluoropentyl group, a 1,1-difluoropentyl group, a 1,2-difluoropentyl group, a 1,3-difluoropentyl group, a 1,4-difluoropentyl group, a 1,5-difluoropentyl group, a 2,2-difluoroperityl group, a 2,3-difluoropentyl group, a 2,4-difluoropentyl group, a 2,5-difluoropentyl group, a 3,3-difluoropentyl group, a 3,4-difluoropentyl group, a 3,5-difluoropentyl group, a 4,5-difluoropentyl group, a 5,5-difluoropentyl group, a 1,1,2-trifluoropentyl group, a 1,1,3-trifluoropentyl group, a 1,1,4-trifluoropentyl group, a 1,1,5-trifluoropentyl group, a 1,2,2-trifluoropentyl group, a 1,2,3-trifluoropentyl group, a 1,2,4-trifluoropentyl group, a 1,2,5-trifluoropentyl group, a 1,3,3-trifluoropentyl group, a 1,3,4-trifluoropentyl group, a 1,3,5-trifluoropentyl group, a 1,4,4-trifluoropentyl group, a 1,4,5-trifluoropentyl group, a 1,5,5-trifluoropentyl group, a 2,2,3-trifluoropentyl group, a 2,2,4-trifluoropentyl group, a 2,2,5-trifluoropentyl group, a 2,3,3-trifluoropentyl group, a 2,3,4-trifluoropentyl group, a 2,3,5-trifluoropentyl group, a 2,4,4-trifluoropentyl group, a 2,4,5-trifluoropentyl group, a 2,5,5-trifluoropentyl group, a 3,3,4-trifluoropentyl group, a 3,3,5-trifluoropentyl group, a 3,4.,4-trifluoropentyl group, a 3,4,5-trifluoropentyl group, a 3,5,5-trifluoropentyl group, a 4,4,5-trifluoropentyl group, a 4,5,5-trifluoropentyl group, a 5,5,5-trifluoropentyl group, a 1,1,2,2-tetrafluoropentyl group, a 1,1,2,3-tetrafluoropentyl group, a 1,1,2,4-tetrafluoropentyl group, a 1,1,2,5-tetrafluoropentyl group, a 1,1,3,3-tetrafluoropentyl group, a 1,1,3,4-tetrafluoropentyl group, a 1,1,3,5-tetrafluoropentyl group, a 1,1,4,4-tetrafluoropentyl group, a 1,1,4,5-tetrafluoropentyl group, a 1,1,5,5-tetrafluoropentyl group, a 1,2,2,3-tetrafluoropentyl group, a 1,2,2,4-tetrafluoropentyl group, a 1,2,2,5-tetrafluoropentyl group, a 1,2,3,3-tetrafluoropentyl group, a 1,2,3,4-tetrafluoropentyl group, a 1,2,3,5-tetrafluoropentyl group, a 1,2,4,4-tetrafluoropentyl group, a 1,2,4,5-tetrafluoropentyl group, a 1,2,5,5-tetrafluoropentyl group, a 1,3,3,4-tetrafluoropentyl group, a 1,3,3,5-tetrafluoropentyl group, a 1,3,4,4-tetrafluoropentyl group, a 1,3,4,5-tetrafluoropentyl group, a 1,3,5,5-tetrafluoropentyl group, a 1,4,4,5-tetrafluoropentyl group, a 1,4,5,5-tetrafluoropentyl group, a 1,5,5,5-tetrafluoropentyl group, a 2,2,3,3-tetrafluoropentyl group, a 2,2,3,4-tetrafluoropentyl group, a 2,2,3,5-tetrafluoropentyl group, a 2,2,4,4-tetrafluoropentyl group, a 2,2,4,5-tetrafluoropentyl group, a 2,2,5,5-tetrafluoropentyl group, a 2,3,3,4-tetrafluoropentyl group, a 2,3,3,5-tetrafluoropentyl group, a 2,3,4,4-tetrafluoropentyl group, a 2,3,4,5-tetrafluoropentyl group, a 2,3,5,5-tetrafluoropentyl group, a 2,4,4,5-tetrafluoropentyl group, a 2,4,5,5-tetrafluoropentyl group, a 2,5,5,5-tetrafluoropentyl group, a 3,3,4,4-tetrafluoropentyl group, a 3,3,4,5-tetrafluoropentyl group, a 3,3,5,5-tetrafluoropentyl group, a 3,4,4,5-tetrafluoropentyl group, a 3,4,5,5-tetrafluoropentyl group, a 3,5,5,5-tetrafluoropentyl group, a 4,4,5,5-tetrafluoropentyl group, a 4,5,5,5-tetrafluoropentyl group, a 1,1,2,2,3-pentafluoropentyl group, a 1,1,2,2,4-pentafluoropentyl group, a 1,1,2,2,5-pentafluoropentyl group, a 1,1,2,3,3-pentafluoropentyl group, a 1,1,2,3,4-pentafluoropentyl group, a 1,1,2,3,5-pentafluoropentyl group, a 1,1,2,4,4-pentafluoropentyl group, a 1,1,2,4,5-pentafluoropentyl group, a 1,1,2,5,5-pentafluoropentyl group, a 1,1,3,3,4-pentafluoropentyl group, a 1,1,3,3,5-pentafluoropentyl group, a 1,1,3,4,4-pentafluoropentyl group, a 1,1,3,4,5-pentafluoropentyl group, a 1,1,3,5,5-pentafluoropentyl group, a 1,1,4,4,5-pentafluoropentyl group, a 1,1,4,5,5-pentafluoropentyl group, a 1,1,5,5,5-pentafluoropentyl group, a 1,2,2,3,3-pentafluoropentyl group, a 1,2,2,3,4-pentafluoropentyl group, a 1,2,2,3,5-pentafluoropentyl group, a 1,2,2,4,4-pentafluoropentyl group, a 1,2,2,4,5-pentafluoropentyl group, a 1,2,2,5,5-pentafluoropentyl group, a 1,2,3,3,4-pentafluoropentyl group, a 1,2,3,3,5-pentafluoropentyl group, a 1,2,3,4,4-pentafluoropentyl group, a 1,2,3,4,5-pentafluoropentyl group, a 1,2,3,5,5-pentafluoropentyl group, a 1,2,4,4,5-pentafluoropentyl group, a 1,2,4,5,5-pentafluoropentyl group, a 1,2,5,5,5-pentafluoropentyl group, a 1,3,3,4,4-pentafluoropentyl group, a 1,3,3,4,5-pentafluoropentyl group, a 1,3,3,5,5-pentafluoropentyl group, a 1,3,4,4,5-pentafluoropentyl group, a 1,3,4,5,5-pentafluoropentyl group, a 1,3,5,5,5-pentafluoropentyl group, a 1,4,4,5,5-pentafluoropentyl group; a 1;4,5,5,5-pentafluoropentyl group, a 2,2,3,3,4-pentafluoropentyl group, a 2,2,3,3,5-pentafluoropentyl group, a 2,2,3,4,4-pentafluoropentyl group, a 2,2,3,4,5-pentafluoropentyl group, a 2,2,3,5,5-pentafluoropentyl group, a 2,2,4,4,5-pentafluoropentyl group, a 2,2,4,5,5-pentafluoropentyl group, a 2,2,5,5,5-pentafluoropentyl group, a 2,3,3,4,4-pentafluoropentyl group, a 2,3,3,4,5-pentafluoropentyl group, a 2,3,3,5,5-pentafluoropentyl group, a 2,3,4,4,5-pentafluoropentyl group, a 2,3,4,5,5-pentafluoropentyl group, a 2,3,5,5,5-pentafluoropentyl group, a 2,4,4,5,5-pentafluoropentyl group, a 2,4,5,5,5-pentafluoropentyl group, a 3,3,4,4,5-pentafluoropentyl group, a 3,3,4,5,5-pentafluoropentyl group, a 3,3,5,5,5-pentafluoropentyl group, a 3,4,5,5,5-pentafluoropentyl group, a 4,4,5,5,5-pentafluoropentyl group, a 1,1,2,2,3,3-hexafluoropentyl group, a 1,1,2,2,3,4-hexafluoropentyl group, a 1,1,2,2,3,5-hexafluoropentyl group, a 1,1,2,2,5,5-hexafluoropentyl group, a 1,1,2,3,3,4-hexafluoropentyl group, a 1,1,2,3,3,5-hexafluoropentyl group, a 1,1,2,3,4,4-hexafluoropentyl group, a 1,1,2,3,4,5-hexafluoropentyl group, a 1,1,2,3,5,5-hexafluoropentyl group, a 1,1,2,4,4,5-hexafluoropentyl group, a 1,1,2,4,5,5-hexafluoropentyl group, a 1,1,2,5,5,5-hexafluoropentyl group, a 1,1,3,3,4,4-hexafluoropentyl group, a 1,1,3,3,4,5-hexafluoropentyl group, a 1,1,3,3,5,5-hexafluoropentyl group, a 1,1,3,4,4,5-hexafluoropentyl group, a 1,1,3,4,5,5-hexafluoropentyl group, a 1,1,3,5,5,5-hexafluoropentyl group, a 1,1,4,4,5,5-hexafluoropentyl group, a 1,1,4,5,5,5-hexafluoropentyl group, a 1,2,2,3,3,4-hexafluoropentyl group, a 1,2,2,3,3,5-hexafluoropentyl group, a 1,2,2,3,4,4-hexafluoropentyl group, a 1,2,2,3,4,5-hexafluoropentyl group, a 1,2,2,3,5,5-hexafluoropentyl group, a 1,2,2,4,4,5-hexafluoropentyl group, a 1,2,2,4,5,5-hexafluoropentyl group, a 1,2,2,5,5,5-hexafluoropentyl group, a 1,2,3,3,4,4-hexafluoropentyl group, a 1,2,3,3,4,5-hexafluoropentyl group, a 1,2,3,3,5,5-hexafluoropentyl group, a 1,2,3,4,4,5-hexafluoropentyl group, a 1,2,3,4,5,5-hexafluoropentyl group, a 1,2,3,5,5,5-hexafluoropentyl group, a 1,2,4,4,5,5-hexafluoropentyl group, a 1.,2,4,5,5,5-hexafluoropentyl group, a 1,3,3,4,4,5-hexafluoropentyl group, a 1,3,3,4,5,5-hexafluoropentyl group, a 1,3,3,5,5,5-hexafluoropentyl group, a 1,3,4,4,5,5-hexafluoropentyl group, a 1,3,4,5,5,5-hexafluoropentyl group, a 1,4,4,5,5,5-hexafluoropentyl group, a 2,2,3,3,4,4-hexafluoropentyl group, a 2,2,3,3,4,5-hexafluoropentyl group, a 2,2,3,3,5,5-hexafluoropentyl group, a 2,2,3,4,4,5-hexafluoropentyl group, a 2,2,3,4,5,5-hexafluoropentyl group, a 2,2,3,5,5,5-hexafluoropentyl group, a 2,2,4,4,5,5-hexafluoropentyl group, a 2,2,4,5,5,5-hexafluoropentyl group, a 2,3,3,4,4,5-hexafluoropentyl group, a 2,3,3,4,5,5-hexafluoropentyl group, a 2,3,3,5,5,5-hexafluoropentyl group, a.2,3,4,4,5,5-hexafluoropentyl group, a 2,3,4,5,5,5-hexafluoropentyl group, a 2,4,4,5,5,5-hexafluoropentyl group, a 3,3,4,4,5,5-hexafluoropentyl group, a 3,3,4,5,5,5-hexafluoropentyl group, a 3,4,4,5,5,5-hexafluoropentyl group, a 1,1,2,2,3,3,4-heptafluoropentyl group, a 1,1,2,2,3,3,5-heptafluoropentyl group, a 1,1,2,2,3,4,4-heptafluoropentyl group, a 1,1,2,2,3,4,5-heptafluoropentyl group, a 1,1,2,2,3,5,5-heptafluoropentyl group, a 1,1,2,2,4,4,5-heptafluoropentyl group, a 1,1,2,2,4,5,5-heptafluoropentyl group, a 1,1,2,2,5,5,5-heptafluoropentyl group, a 1,1,2,3,3,4,4-heptafluoropentyl group, a 1,1,2,3,3,4,5-heptafluoropentyl group, a 1,1,2,3,3,5,5-heptafluoropentyl group, a 1,1,2,3,4,4,5-heptafluoropentyl group, a 1,1,2,3,4,5,5-heptafluoropentyl group, a 1,1,2,3,5,5,5-heptafluoropentyl group, a 1,1,2,4,4,5,5-heptafluoropentyl group, a 1,1,2,4,5,5,5-heptafluoropentyl group, a 1,1,3,3,5,5,5-heptafluoropentyl group, a 1,1,3,4,4,5,5-heptafluoropentyl group, a 1,1,3,4,5,5,5-heptafluoropentyl group, a 1,1,4,4,5,5,5-heptafluoropentyl group, a 1,2,2,3,3,4,4-heptafluoropentyl group, a 1,2,2,3,3,4,5-heptafluoropentyl group, a 1,2,2,3,3,5,5-heptafluoropentyl group, a 1,2,2,3,4,4,5-heptafluoropentyl group, a 1,2,2,3,4,5,5-heptafluoropentyl group, a 1,2,2,3,5,5,5-heptafluoropentyl group, a 1,2,2,4,4,5,5-heptafluoropentyl group, a 1,2.,2,4,5,5,5-heptafluoropentyl group, a 1,2,3,3,4,4,5-heptafluoropentyl group, a 1,2,3,3,4,5,5-heptafluoropentyl group, a 1,2,3,3,5,5,5-heptafluoropentyl group, a 1,2,3,4,4,5,5-heptafluoropentyl group, a 1,2,3,4,5,5,5-heptafluoropentyl group, a 1,2,4,4,5,5,5-heptafluoropentyl group, a 1,3,3,4,4,5,5-heptafluoropentyl group, a 1,3,3,4,5,5,5-heptafluoropentyl group, a 1,3,4,4,5,5,5-heptafluoropentyl group, a 2,2,3,3,4,4,5-heptafluoropentyl group, a 2,2,3,3,4,5,5-heptafluoropentyl group, a 2,2,3,3,5,5,5-heptafluoropentyl group, a 2,2,3,4,4,5,5-heptafluoropentyl group, a 2,2,3,4,5,5,5-heptafluoropentyl group, a 2,2,4,4,5,5,5-heptafluoropentyl group, a 2,3,3,4,4,5,5-heptafluoropentyl group, a 2,3,3,4,5,5,5-heptafluoropentyl group, a 2,3,4,4,5,5,5-heptafluoropentyl group, a 3,3,4,4,5,5,5-heptafluoropentyl group, a 1,1,2,2,3,3,4,4-octafluoropentyl group, a 1,1,2,2,3,3,4,5-octafluoropentyl group, a 1,1,2,2,3,3,5,5-octafluoropentyl group, a 1,1,2,2,3,4,4,5-octafluoropentyl group, a, 1,1,2,2,3,4,5,5-octafluoropentyl group, a 1,1,2,2,3,5,5,5-octafluoropentyl group, a 1,1,2,2,4,4,5,5-octafluoropentyl group, a 1,1,2,2,4,5,5,5-octafluoropentyl group, a 1,1,2,3,3,4,4,5-octafluoropentyl group, a 1,1,2,3,3,4,5,5-octafluoropentyl group, a 1,1,2,3,3,5,5,5-octafluoropentyl group, a 1,1,2,3,4,4,5,5-octafluoropontyl group, a 1,1,2,3,4,5,5,5-octafluoropentyl group, a 1,1,2,4,4,5,5,5-octafluoropentyl group, a 1,1,3,3,4,4,5,5-octafluoropentyl group, a 1,1,3,3,4,5,5,5-octafluoropentyl group, a 1,1,3,4,4,5,5,5-octafluoropentyl group, a 1,2,2,3,3,4,4,5-octafluoropentyl group, a 1,2,2,3,3,4,5,5-octafluoropentyl group, a 1,2,2,3,3,5,5,5-octafluoropentyl group, a 1,2,2,3,4,4,5,5-octafluoropentyl group, a 1,2,2,3,4,5,5,5-octafluoropentyl group, a 1,2,2,4,4,5,5,5-octafluoropentyl group, a 1,2,3,3,4,4,5,5-octafluoropentyl group, a 1,2,3,3,4,5,5,5-octafluoropentyl group, a 1,2,3,4,4,5,5,5-octafluoropentyl group, a 1,3,3,4,4,5,5,5-octafluoropentyl group, a 2,2,3,3,4,4,5,5-octafluoropentyl group, a 2,2,3,3,4,5,5,5-octafluoropentyl group, a 2,2,3,4,4,5,5,5-octafluoropentyl group, a 2,3,3,4,4,5,5,5-octafluoropentyl group, a 1,1,2,2,3,3,4,4,5-nonafluoropentyl group, a 1,1,2,2,3,3,4,5,5-nonafluoropentyl group, a 1,1,2,2,3,3,5.,5,5-nonafluoropentyl group, a 1,1,2,2,3,4,4,5,5-nonafluoropentyl group, a 1,1,2,2,3,4,5,5,5-nonafluoropentyl group, a 1,1,2,2,4,4,5,5,5-nonafluoropentyl group, a 1,1,2,3,3,4,4,5,5-nonafluoropentyl group, a 1,1,2,3,3,4,5,5,5-nonafluoropentyl group, a 1,1,2,3,4,4,5,5,5-nonafluoropentyl group, a 1,1,3,3,4,4,5,5,5-nonafluoropentyl group, a 1,2,2,3,3,4,4,5,5-nonafluoropentyl group, a 1,2,2,3,3,4,5,5,5-nonafluoropentyl group, a 1,2,2,3,4,4,5,5,5-nonafluoropentyl group, a 1,2,3,3,4,4,5,5,5-nonafluoropentyl group, a 2,2,3,3,4,4,5,5,5-nonafluoropentyl group, a 1,1,2,2,3,3,d,4,5,5-decafluoropentyl group, a 1,1,2,2,3,3,4,5,5,5-decafluoropentyl group, a 1,1,2,2,3,4,4,5,5,5-decafluoropentyl group, a 1,1,2,3,3,4,4,5,5,5-decafluoropentyl group, a 1,2,2,3,3,4,4,5,5,5-decafluoropentyl group and an undecafluoropentyl group.

Among them, C1 to C5 perfluoroalkyl groups are preferable.

The C1 to C5 perfluoroalkyl group includes a trifluoromethyl group, a pentafluoroethyl group, a heptafluoropropyl group, a nonafluorobutyl group and an undecafluoropentyl group, and among them, a trifluoromethyl group is preferable.

Examples of the olefin (II) include 3-fluoropropene, 3,3-difluoropropene, 3,3,3-trifluoropropene, 3-fluoro-1-butene, 4-fluoro-1-butene, 3,3-difluoro-1-butene, 3,4-difluoro-1-butene, 4,4-difluoro-1-butene, 3,3,4-trifluoro-1-butene, 3,4,4-trifluoro-1-butene, 4,4,4-trifluoro-1-butene, 3,3,4,4-tetrafluoro-1-butene, 3,4,4,4-tetrafluoro-1-butene, 3,3,4,4,4-pentafluoro-1-butene, 4,4,4-trifluord-3-trifluoromethyl-1-butene, 3-fluoro-1-pentene, 4-fluoro-1-pentene, 5-fluoro-1-pentene, 3,3-difluoro-1-pentene, 3,4-difluoro-1-pentene, 4,4-difluoro-1-pentene, 4,5-difluoro-1-pentene, 5,5-difluoro-1-pentene, 3,3,4-trifluoro-1-pentene, 3,3,5-trifluoro-1-pentene, 3,4,4-trifluoro-1-pentene, 3,4,5-trifluoro-1-pentene, 3,5,5-trifluoro-1-pentene, 4,4,5-trifluoro-1-pentene, 5,5,5-trifluoro-1-pentene, 3,3,4,4-tetrafluoro-1-pentene, 3,3,4,5-tetrafluoro-1-pentene, 3,3,5,5-tetrafluoro-1-pentene, 3,4,4,5-tetrafluoro-1-pentene, 3,4,8,5-tetraflucro-1-pentene, 3,5,5,5-tetrafluoro-1-pentene, 4,4,5,5-tetrafluoro-1-pentene, 4,5,5,5-tetrafluoro-1-pentene, 3,3,4,4,5-pentafluoro-1-pentene, 3,3,4,5,5-pentafluoro-1-pentene, 3,3,5,5,5-pentafluoro-1-pentene, 3,4,4,5,5-pentafluoro-1-pentene, 3,4,5,5,5-pentafluoro-1-pentene, 4,4,5,5,5-pentafluoro-1-pentene, 3,3,4,4,5,5-hexafluoro-1-pentene, 3,3,4,5,5,5-hexafluoro-1-pentene, 3,4,4,5,5,5-hexafluoro-1-pentene, 3,3,4,4,5,5,5-heptafluoro-1-pentene, 3-fluoro-1-hexene, 4-fluoro-1-hexene, 5-fluoro-1-hexen 6-fluoro-1-hexene, 3,3-difluoro-1-hexene, 3,4-difluoro-1-hexene, 4,4-difluoro-1-hexene, 4,5-difluoro-1-hexene, 5,5-difluoro-1-hexene, 5,6-difluoro-1-hexene, 6,6-difluoro-1-hexene, 3,3,4-trifluoro-1-hexene, 3,3,5-trifluoro-1-hexene, 3,3,6-trifluoro-1-hexene, 3,4,4-trifluoro-1-hexene, 3,4,5-trifluoro-1-hexene, 3,4,6-trifluoro-1-hexene, 3,5,5-trifluoro-1-hexene, 3,5,6-trifluoro-1-hexene, 3,6,6-trifluoro-1-hexene, 4,4,5-trifluoro-1-hexene, 4,4,6-trifluoro-1-hexene, 4,5,5-trifluoro-1-hexene, 4,5,6-trifluoro-1-hexene, 4,6,6-trifluoro-1-hexene, 5,5,6-trifluoro-1-hexene, 5,6,6-trifluoro-1-hexene, 6,6,6-trifluoro-1-hexene, 3,3,4,4-tetrafluoro-1-hexene, 3,3,4,5-tetrafluoro-1-hexene, 3,3,4,6-tetrafluoro-1-hexene, 3,3,5,5-tetrafluoro-1-hexene, 3,3,5,6-tetrafluoro-1-hexene, 3,3,6,6-tetrafluoro-1-hexene, 3,4,4,5-tetrafluoro-1-hexene, 3,4,4,6-tetrafluoro-1-hexene, 3,4,5,5-tetrafluoro-1-hexene, 3,4,5,6-tetrafluoro-1-hexene, 3,4,6,6-tetrafluoro-1-hexene, 3,5,5,6-tetrafluoro-1-hexene, 3,5,6,6-tetrafluoro-1-hexene, 3,6,6,6-tetrafluoro-1-hexene, 4,4,5,5-tetrafluoro-1-hexene, 4,4,5,6-tetrafluoro-1-hexene, 4,4,6,6-tetrafluoro-1-hexene, 4,5,5,6-tetrafluoro-1-hexene, 4,5,6,6-tetrafluoro-1-hexene, 4,6,6,6-tetrafluoro-1-hexene, 5,5,6,6-tetrafluoro-1-hexene, 5,6,6,6-tetrafluoro-1-hexene, 3,3,4,4,5-pentafluoro-1-hexene, 3,3,4,4,6-pentafluoro-1-hexene, 3,3,4,5,5-pentafluoro-1-hexene, 3,3,4,5,6-pentafluoro-1-hexene, 3,3,4,6,6-pentafluoro- 1-hexene, 3,3,5,5,6-pentafluoro-1-hexene, 3,3,5,6,6-pentafluoro-1-hexene, 3,3,6,6,6-pentafluoro-1-hexene, 3,4,4,5,5-pentafluoro-1-hexene, 3,4,4,5,6-pentafluoro-1-hexene, 3,4,4,6,6-pentafluoro-1-hexene, 3,4,5,5,6-pentafluoro-1-hexene, 3,4,5,6,6-pentafluoro-1-hexene; 3,4,6,6,6-pentafluoro-1-hexene, 3,5,5,6,6-pentafluoro-1-hexene, 3,5,6,6,6-pentafluoro-1-hexene, 4,4,5,5,6-pentafluoro-1-hexene, 4,4,5,6,6-pentafluoro-1-hexene, 4,4,6,5,6-pentafluoro-1-hexene, 4,5,5,6,6-pentafluoro-1-hexene, 4,5,6,6,6-pentafluoro-1-hexene, 5,5,6,6,6-pentafluoro-1-hexene, 3,3,4,4,5,5-hexafluoro-1-hexene, 3,3,4,4,5,6-hexafluoro-1-hexene, 3,3,4,4,6,6-hexafluoro-1-hexene, 3,3,4,5,5,6-hexafluoro-1-hexene, 3,3,4,5,6,6-hexafluoro-1-hexene, 3,3,4,6,6,6-hexafluoro-1-hexene, 3,3,5,5,6,6-hexafluoro-1-hexene, 3,3,5,6,6,6-hexafluoro-1-hexene, 3,4,4,5,5,6-hexafluoro-1-hexene, 3,4,4,5,6,6-hexafluoro-1-hexene, 3,4,4,6,6,6-hexafluoro-1-hexene, 3,4,5,5,6,6-hexafluoro-1-hexene, 3,4,5,6,6,6-hexafluoro-1-hexene, 3,5,5,6,6,6-hexafluoro-1-hexene, 4,5,5,6,6,6-hexafluoro-1-hexene, 3,3,4,4,5,5,6-heptafluoro-1-hexene, 3,3,4,4,5,6,6-heptafluoro-1-hexene, 3,3,4,4,6,6,6-heptafluoro-1-hexene, 3,3,4,5,5,6,6-heptafluoro-1-hexene, 3,3,4,5,6,6,6-heptafluoro-1-hexene, 3,3,5,5,6,6,6-heptafluoro-1-hexene, 3,4,4,5,5,6,6-heptafluoro-1-hexene, 3,4,4,5,6,6,6-heptafluoro-1-hexene, 3,4,5,5,6,6,6-heptafluoro-1-hexene, 4,4,5,5,6,6,6-heptafluoro-1-hexene, 3,3,4,4,5,5,6,6-octafluoro-1-hexene, 3,3,4,4,5,6,6,6-octafluoro-1-hexene, 3,3,4,5,5,6,6,6-ootafluoro-1-hexene, 3,4,4,5,5,6,6,6-octafluoro-1-hexene, 3,3,4,4,5,5,6,6,6-nonafluoro-1-hexene, 3-flubro-1-heptene, 4-fluoro-1-heptene, 5-fluoro-1-heptene, 6-fluoro-1-heptene, 7-fluoro-1-heptene, 3,3,-difluoro-1-heptene, 3,4,-difluoro-1-heptene, 4,4-difluoro-1-heptene, 4,5-difluoro-1-heptene, 5,5-difluoro-1-heptene, 5,6-difluoro-1-heptene, 6,6-difluoro-1-heptene, 6,7-difluoro-1-heptene, 7,7-difluoro-1-heptene, 3,3,4-trifluoro-1-heptene, 3,3,5-trifluoro-1-heptene, 3,3,6-trifluoro-1-heptene, 3,3,7-trifluoro-1-heptene, 3,4,4-trifluoro-1-heptene, 3,4,5-trifluoro-1-heptene, 3,4,6-trifluoro-1-heptene, 3,4,7-trifluoro-1-heptene, 3,5,5-trifluoro-1-heptene, 3,5,6-trifluoro-1-heptene, 3,5,7-trifluoro-1-heptene, 3,6,6-trifluoro-1-heptene, 3,5,7-trifluoro-1-heptene, 3,7,7-trifluoro-1-heptene, 4,4,5-trifluoro-1-heptene, 4,4,6-trifluoro-1-heptene, 4,4,7-trifluoro-1-heptene, 4,5,5-trifluoro-1-heptene, 4,5,6-trifluoro-1-heptene, 4,5,7-trifluoro-1-heptene, 4,6,6-trifluoro-1-heptene, 4,6,7-trifluoro-1-heptene, 4,7,7-trifluoro-1-heptene, 5,5,6-tri.fluoro-1-heptene, 5,5,7-trifluoro-1-heptene, 5,6,6-trifluoro-1-heptene, 5,6,7-trifluoro-1-heptene, 5,7,7-trifluoro-1-heptene, 6,6,7-trifluoro-1-heptene, 6,7,7-tri.fluoro-1-heptene, 7,7,7-trifluoro-1-heptene, 3,3,4,4-tetrafluoro-1-heptene, 3,3,4,5-tetrafluoro-1-heptene, 3,3,4,6=tetrafluoro-1-heptene, 3,3,4,7-tetrafluoro-1-heptene, 3,3,5,5-tetrafluoro-1-heptene, 3,3,5,6-tetrafluoro-1-heptene, 3,3,5,7-tetrafluoro-1-heptene, 3,3,6,6-tetrafluoro-1-heptene, 3,3,6,7-tetrafluoro-1-heptene, 3,3,7,7-tetrafluoro-1-heptene, 3,4_{;}4,5-tetrafluoro-1-heptene, 3,4,4,6-tetrafluoro-1-heptene, 3,4,4,7-tetrafluoro-1-heptene, 3,4,5,5-tetrafluoro-1-heptene, 3,4,5,6-tetrafluoro-1-heptene, 3,4,5,7-tetrafluoro-1-heptene, 3,4,6,6-tetrafluoro-1-heptene, 3,4,6,7-tetrafluoro-1-heptene, 3,4,7,7-tetrafluoro-1-heptene, 3,5,5,6-tetrafluoro-1-heptene, 3,5,5,7-tetrafluoro-1-heptene, 3,6,6,7-tetrafluoro-1-heptene, 3,6,7,7-tetrafluoro-1-heptene, 3,7,7,7-tetrafluoro-1-heptene, 4,4,5,5-tetrafluoro-1-heptene, 4,4,5,6-tetrafluoro-1-heptene, 4,4,5,7-tetrafluoro-1-heptene, 4,4,6,6-tetrafluoro-1-heptene, 4,4,6,7-tetrafluoro-1-heptene, 4,4,7,7-tetrafluoro-1-heptene, 4,5,5,6-tetrafluoro-1-heptene, 4,5,5,7-tetrafluoro-1-heptene, 4,5,6,6-tetrafluoro-1-heptene, 4,5,6,7-tetrafluoro-1-heptene, 4,5,7,7-tetrafluoro-1-heptene, 4,6,6,7-tetrafluoro-1-heptene, 4,6,7,7-tetrafluoro-1-heptene, 4,7,7,7-tetrafluoro-1-heptene, 5,5,6,6-tetrafluoro-1-heptene, 5,5,6,7-tetrafluoro-1-heptene, 5,5,7,7-tetrafluoro-1-heptene, 5,6,6,7-tetrafluoro-1-heptene, 5,6,7,7-tetrafluoro-1-heptene, 5,7,7,7-tetrafluoro-1-heptene, 6,6,7,7-tetrafluoro-1-heptene, 6,7,7,7-tetrafluoro-1-heptene, 3,3,4,4,5-pentafluoro-1-heptene, 3,3,4,4,6-pentafluoro-1-heptene, 3,3,4,4,7-pentafluoro-1-heptene, 3,3,4,5,5-pentafluoro-1-heptene, 3,3,4,5,6-pentafluoro-1-heptene, 3,3,4,5,7-pentafluoro-1-heptene, 3,3,4,6,6-pentafluoro-1-heptene, 3,3,4,6,7-pentafluoro-1-heptene, 3,3,4,7,7-pentafluoro-1-heptene, 3,3,5,5,6-pentafluoro-1-heptene, 3,3,5,5,7-pentafluoro-1-heptene, 3,3,5,6,6-pentafluoro-1-heptene, 3,3,5,6,7-pentafluoro-1-heptene, 3,3,5,7,7-pentafluoro-1-heptene, 3,3,6,6,7-pentafluoro-1-heptene, 3,3,6,7,7-pentafluoro-1-heptene, 3,3,7,7,7-pentafluoro-1-heptene, 3,4,4,5,5-pentafluoro-1-heptene, 3,4,4,5,6-pentafluoro-1-heptene, 3,4,4,5,7-pentafluoro-1-heptene, 3,4,4,6,6-pentafluoro-1-heptene, 3,4,4,6,7-pentafluoro-1-heptene, 3,4,4,7,7-pentafluoro-1-heptene, 3,4,5,5,6-pentafluoro-1-heptene, 3,4,5,5,7-pentafluoro-1-heptene, 3,4,5,6,6-pentafluoro-1-heptene, 3,4,5,6,7-pentafluoro-1-heptene, 3,4,5,7,7-pentafluoro-1-heptene, 3,4,6,6,7-pentafluoro-1-heptene, 3,4,6,7,7-pentafluoro-1-heptene, 3,4,7,7,7-pentafluoro-1-heptene, 3,5,5,6,6-pentafluoro-1-heptene, 3,5,5,6,7-pentafluoro-1-heptene, 3,5,5,7,7-pentafluoro-1-heptene, 3,5,6,6,7-pentafluoro-1-heptene, 3,5,6,7,7-pentafluoro-1-heptene, 3,5,7,7,7-pentafluoro-1-heptene, 3,6,6,7,7-pentafluoro-1-heptene, 3,6,7,7,7-pentafluoro-1-heptene, 4,4,5,5,6-pentafluoro-1-heptene, 4,4,5,5,7-pentafluoro-1-heptene, 4,4,5,5,6-pentafluoro-1-heptene, 4,4,5,6,7-pentafluoro-1-heptene, 4,4,5,7,7-pentafluoro-1-heptene, 4,4,6,6,7-pentafluoro-1-heptene, 4,4,6,7,7-pentafluoro-1-heptene, 4,4,7,7,7-pentafluoro-1-heptene, 4,5,5,6,6-pentafluoro-1-heptene, 4,5,5,6,7-pentafluoro-1-heptene, 4,5,5,7,7-pentafluoro-1-heptene, 4,5,6,6,7-pentafluoro-1-heptene, 4,5,6,7,7-pentafluoro-1-heptene, 4,5,7,7,7-pentafluoro-1-heptene, 4,6,6,7,7-pentafluoro-1-heptene, 4,6,7,7,7-pentafluoro-1-heptene, 5,5,6,6,7-pentafluoro-1-heptene, 5,5,6,7,7-pentafluoro-1-heptene, 5,5,7,7,7-pentafluoro-1-heptene, 5,6,6,7,7-pentafluoro-1-heptene, 5,6,7,7,7-pentafluoro-1-heptene, 6,6,7,7,7-pentafluoro-1-heptene, 3,3,4,4,5,5-hexafluoro-1-heptene, 3,3,4,4,5,6-hexafluoro-1-heptene, 3,3,4,4,5,7-hexafluoro-1-heptene, 3,3,4,4,6,6-hexafluoro-1-heptene, 3,3,4,4,6,7-hexafluoro-1-heptene, 3,3,4,4,7,7-hexafluoro-1-heptene, 3,3,4,5,5,6-hexafluoro-1-heptene, 3,3,4,5,5,7-hexafluoro-1-heptene, 3,3,4,5,6,6-hexafluoro-1-heptene, 3,3,4,5,6,7-hexafluoro-1-heptene, 3;3,4,5,7,7-hexafluoro-1-heptene, 3,3,4,6,6,7-hexafluoro-1-heptene, 3,3,4,6,7,7-hexafluoro-1-heptene, 3,3,4,7,7,7-hexafluoro-1-heptene, 3,3,5,5,6,6-hexafluoro-1-heptene, 3,3,5,5,6,7-hexafluoro-1-heptene, 3,3,5,5,7,7-hexafluoro-1-heptene, 3,3,5,6,6,7-hexafluoro-1-heptene, 3,3,5,6,7,7-hexafluoro-1-heptene, 3,3,5,7,7,7-hexafluoro-1-heptene, 3,3,6,6,7,7-hexafluoro-1-heptene, 3,3,6,7,7,7-hexafluoro-1-heptene, 3,4,4,5,5,6-hexafluoro-1-heptene, 3,4,4,5,5,7-hexafluoro-1-heptene, 3,4,4,5,5,6-hexafluoro-1-heptene, 3,4,4,5,6,7-hexaf1uoro-1-heptene, 3,4,4,5,7,7-hexafluoro-1-heptene, 3,4,4,6,6,7-hexafluoro-1-heptene, 3,4,4,6,7,7-hexafluoro-1-heptene, 3,4,4,7,7,7-hexafluoro-1-heptene, 3,4,5,5,6,6-hexafluoro-1-heptene, 3,4,5,5,6,7-hexafluoro-1-heptene, 3,4,5,5,7,7-hexafluoro-1-heptene, 3,4,5,6,6,7-hexafluoro-1-heptene, 3,4,5,6,7,7-hexafluoro-1-heptene, 3,4,5,7,7,7-hexafluoro-1-heptene, 3,4,6,6,7,7-hexafluoro-1-heptene, 3,4,6,7,7,7-hexafluoro-1-heptene, 3,5,5,6,6,7-hexafluoro-1-heptene, 3,5,5,6,7,7-hexafluoro-1-heptene, 3,5,5,7,7,7-hexafluoro-1-heptene, 3,5,6,6,7,7-hexafluoro-1-heptene, 3,5,6,7,7,7-hexafluoro-1-heptene, 3,6,6,7,7,7-hexafluoro-1-heptene, 4,4,5,5,6,6-hexafluoro-1-heptene, 4,4,5,5,6,7-hexafluoro-1-heptene, 4,4,5,5,7,7-hexafluoro-1-heptene, 4,4,5,6,6,7-hexafluoro-1-heptene, 4,4,5,6,7,7-hexafluoro-1-heptene, 4,4,5,7,7,7-hexafluoro-1-heptene, 4,4,6,6,7,7-hexafluoro-1-heptene, 4,4,6,7,7,7-hexafluoro-1-heptene, 4,5,5,6,6,7-hexafluoro-1-heptene, 4,5,5,6,7,7-hexafluoro-1-heptene, 4,5,5,7,7,7-hexafluoro-1-heptene, 4,5,6,6,7,7-hexafluoro-1-heptene, 4,5,6,7,7,7-hexafluoro-1-heptene, 4,6,6,7,7,7-hexafluoro-1-heptene, 5,5,6,6,7,7-hexafluoro-1-heptene, 5,6,6,7,7,7-hexafluoro-1-heptene, 3,3,4,4,5,5,6-heptafluoro-1-heptene, 3,3,4,4,5,5,7-heptafluoro-1-heptene, 3,3,4,4,5,6,6-heptafluoro-1-heptene, 3,3,4,4,5,6,7-heptafluoro-1-heptene, 3,3,4,4,5,7,7-heptafluoro-1-heptene, 3,3,4,4,6,6,7-heptafluoro-1-heptene, 3,3,4,4,6,7,7-heptafluoro-1-heptene, 3,3,4,4,7,7,7-heptafluoro-1-heptene, 3,3,4,5,5,6,6-heptafluoro-1-heptene, 3,3,4,5,5,6,7-heptafluoro-1-heptene, 3,3,4,5,5,7,7-heptafluoro-1-heptene, 3,3,4,5,6,6,7-heptafluoro-1-heptene, 3,3,4,5,6,7,7-heptafluoro-1-heptene, 3,3,4,5,7,7,7-heptafluoro-1-heptene, 3,3,4,6,6,7,7,-heptafluoro-1-heptene, 3,3,4,6,7,7,7-heptafluoro-1-heptene, 3,3,5,5,6,6,7-heptafluoro-1-heptene, 3,3,5,5,6,7,7-heptafluoro-1-heptene, 3,3,5,5,7,7,7-heptafluoro-1-heptene, 3,3,5,6,6,7,7-heptafluoro-1-heptene, 3,3,5,6,7,7,7-heptafluoro-1-heptene, 3,3,6,6,7,7,7-heptafluoro-1-heptene, 3,4,4,5,5,6,6-heptafluoro-1-heptene, 3,4,4,5,5,6,7-heptafluoro-1-heptene, 3,4,4,5,5,7,7-heptafluoro-1-heptene, 3,4,4,5,6,6,7-heptafluoro-1-heptene, 3,9,4,5,6,7,7-heptafluoro-1-heptene, 3,4,4,5,7,7,7-heptafluoro-1-heptene, 3,4,4,6,6,7,7-tieptafluoro-1-heptene, 3,4,4,6,7,7,7-heptafluoro-1-heptene, 3,4,5,5,6,6,7-heptafluoro-1-heptene, 3,4,5,5,6,7,7-heptafluoro-1-heptene, 3,4,5,5,7,7,7-heptafluoro-1-heptene, 3,4,5,6,6,7,7-heptafluoro-1-heptene, 3,4,5,6,7,7,7-heptafluoro-1-heptene, 3,4,6,6,7,7,7-heptafluoro-1-heptene; 3,5,5,6,6,7,7-heptafluoro-1-heptene, 3,5,5,6,7,7,7-heptafluoro-1-heptene, 3,5,6,6,7,7,7-heptafluoro-1-heptene, 4,4,5,5,6,6,7-heptafluoro-1-heptene, 4,4,5,5,6,7,7-heptafluoro-1-heptene, 4,4,5,5,7,7,7-heptafluoro-1-heptene, 4,4,5,6,6,7,7-heptafluoro-1-heptene, 4,4,5,6,7,7,7-heptafluoro-1-heptene, 4,4,6,6,7,7,7-heptafluoro-1-heptene, 4,5,5,6,6,7,7-heptafluoro-1-heptene, 4,5,5,6,7,7,7-heptafluoro-1-heptene, 4,5,6,6,7,7,7-heptafluoro-1-heptene, 5,5,6,6,7,7,7-heptafluoro-1-heptene, 3,3,4,4,5,5,6,6-octafluoro-1-heptene, 3,3,4,4,5,5,6,7-octafluoro-1-heptene, 3,3,4,4,5,5,7,7-octafluoro-1-heptene, 3,3,4,4,5,6,6,7-octafluoro-1-heptene, 3,3,4,4,5,6,7,7-octafluoro-1-heptene, 3,3,4,4,5,7,7,7-octafluoro-1-heptene, 3,3,4,4,6,6,7,7-octafluoro-1-heptene, 3,3,4,4,6,7,7,7-octafluoro-1-heptene, 3,3,4,5,5,6,6,7-octafluoro-1-heptene, 3,3,4,5,5,6,7,7-octafluoro-1-heptene, 3,3,4,5,5,7,7,7-octafluoro-1-heptene, 3,3,4,5,6,6,7,7-octafluoro-1-heptene, 3,3,4,5,6,7,7,7-octafluoro-1-heptene, 3,3,4,6,6,7,7,7-octafluoro-1-heptene, 3,3,5,5,6,6,7,7-octafluoro-1-heptene, 3,3,5,5,6,7,7,7-octafluoro-1-heptene, 3,3,5,6,6,7,7,7-octafluoro-1-heptene, 3,4,4,5,5,6,6,7-octafluoro-1-heptene, 3,4,4,5,5,6,7,7-octafluoro-1-heptene, 3,4,4,5,5,7,7,7-octafluoro-1-heptene, 3,4,4,5,6,6,7,7-octafluoro-1-heptene, 3,4,4,5,6,7,7,7-octafluoro-1-heptene, 3,4,4,6,6,7,7,7-octafluoro-1-heptene, 3,4,5,5,6,6,7,7-octafluoro-1-heptene, 3,4,5,5,6,7,7,7-octafluoro-1-heptene, 3,4,5,6,6,7,7,7-octafluoro-1-heptene, 3,5,5,6,6,7,7,7-octafluoro-1-heptene, 4,4,5,5,6,6,7,7-octafluoro-1-heptene, 4,4,5,5,6,7,7,7-octafluoro-1-heptene, 4,4,5,6,6,7,7,7-octafluoro-1-heptene, 4,5,5,6;6,7,7,7-octafluoro-1-heptene, 3,3,4,4,5,5,6,6,7-nonafluoro-1-heptene, 3,3,4,4,5,5,6,7,7-nonafluoro-1-heptene, 3,3,4,4,5,5,7,7,7-nonafluoro-1-heptene, 3,3,4,4,5,6,6,7,7-nonafluoro-1-heptene, 3,3,4,4,5,6,7,7,7-nonafluoro-1-heptene, 3,3,4,4,6,6,7,7,7-nonafluoro-1-heptene, 3,3,4,5,5,6,6,7,7-nonafluoro-1-heptene, 3,3,4,5,5,6,7,7,7-nonafluoro-1-heptene, 3,3,4,5,6,6,7,7,7-nonafluoro-1-heptene, 3,3,5,5,6,6,7,7,7-nonafluoro-1-heptene, 3,4,4,5,5,6,6,7,7-nonafluoro-1-heptene, 3,4,4,5,5,6,7,7,7-nonafluoro-1-heptene, 3,4,4,5,6,6,7,7,7-nonafluoro-1-heptene, 3,4,5,5,6,6,7,7,7-nonafluoro-1-heptene, 4,4,5,5,6,6,7,7,7-nonafluoro-1-heptene, 3,3,4,4,5,5,6,6,7,7-decafluoro-1-heptene, 3,3,4,4,5,5,6,7,7,7-decafluoro-1-heptene, 3,3,4,4,5,6,6,7,7,7-decafluoro-1-heptene, 3,3,4,5,5,6,6,7,7,7-decafluoro-1-heptene, 3,4,4,5,5,6,6,7,7,7-decafluoro-1-heptene, and 3,3,4,4,5,5,6,6,7,7,7-undecafluoro-1-heptene.

Among them, in formula (II) of 3,3,3-trifluoropropene, 3,3,4,4,4-pentafluoro-1-butene and 3,3,4,4,5,5,5-heptafluoro-1-pentene, a fluoroolefin wherein R² is a C1-C5 perfluoroalkyl group is preferable, and 3,3,3-trifluoropropene is more preferable.

A thiocarboxylic acid S-(fluoroalkyl) ester represented by formula (III) 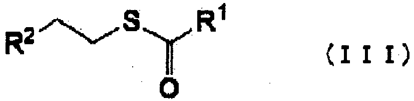 (wherein R¹ and R² have the same meaning as defined above) (hereinafter referred to as ester (III)) can be produced by reacting a thiocarboxylic acid (I) with an olefin (II) in the presence of a radical generator.

The amount of the olefin (II) used in the reaction is usually 0.5 to 10 mol, and preferably 1 to 5 mol, based on 1 mol of the thiocarboxylic acid (I).

Examples of the radical generator include boron-containing compounds such as triethylborane; peroxides such as benzoyl peroxide, tert-butyl hydroperoxide, and hydrogen peroxide; and azo compounds. Among them, azo compounds are preferable, and an azo compound represented by formula (IV)

**R³-N-N-R⁴** (IV)

(wherein R³ and R⁴ are the same or di fferent, and represent a C2-C12 alkyl group that is optionally substituented or carbamoyl group) is more preferable.

Examples of the C2-C12 alkyl group represented by R³ and R⁴ includes an ethyl group, a propyl group, an isopropyl group, a butyl group, an isobutyl group, a pentyl group, a hexyl group, a heptyl group, an octyl group, a nonyl group, a decyl group, an undecyl group, and a dodecyl group. The C2-C12 alkyl group is optionally substituted, and examples of the substituent includes a cyano group; C1-C12 alkoxy groups such as a methoxy group, an ethoxy group, a propoxy group, an isopropoxy group, a butoxy group, an isobutoxy group, a pentyloxy group, a hexyloxy group, a heptyloxy group, an octyloxy group, a nonyloxy group, a decyloxy group, an undecyloxy group and a dodecyloxy group; and a carbamoyl group, a hydroxyl group, an amidino group, an imidazoline-2-yl group and a pyrrolidino group that can have a C1-C12 alkyl group or groups.

Examples of the azo compound represented by formula (IV) include 2,2'-azobisisobutyronitrile, 2,2'-azobis(2,4-dimethyl-4-methoxy)valeronitrile, 2,2'-azobis[2-(2-imidazolin-2-yl)propane] hydrochloride, 2,2'-azobis[2-(2-imidazolin-2-yl)propane] sulfate, 2,2'-azobis(2-methylpropionamidine) dihydrochloride, 2,2'-azobis[N-(2-carboxyethyl)-2-methylpropionamide], 2,2'-azobls{2-[1-(2-hydroxymethyl)-2-imidazolin-2-yl]propane} hydrochloride,2,2'-azobis[2-(2-imidazolin-2-yl)propane], 2,2'-azobis(1-imino-1-pyrrolidino-2-methylpropane) hydrochloride,2,2'-azobis{2-methyl-N-[1,1-bis (hydroxymethyl)-2-hydroxyethyl]propanamide}, 2,2'-azobis[2-methyl-N-(2-hydroxyethyl)methylpropionamide], 2,2'-azobis(2,4-dimethylvaleronitrile), dimethyl-2,2'-azobis(2,-methylpropionate), 2,2'-azobis(2-methylbutyronitrile), 1,1'-azobis(cyclohexane-1-carbonitrile), 2,2'-azobis[N-(2-propenyl)-2-methylpropionamide], 1-[(1-cyano-1-methylethyl)azo]formamide, 2,2'-azobis(N-butyl-2-methylpropionamide) and 2,2'-azobis(N-cyclohexyl-2-methylpropionamide). Among them, 2,2'-azobis(2,4-dimethyl-4-methoxy)valeronitrile is preferable.

The amount of the radical generator used in the reaction is usually from 0.001 to 1 mol, and preferably from 0.01 to 0.1 mol, based on 1 mol of the thiocarboxylic acid (I).

The reaction of the thiocarboxylic acid (I) with the olefin (II) of the present invention (hereinafter referred to as the present reaction) maybe carried out in the presence of a solvent, or may be carried out without solvent.

Examples of the solvent used in the present reaction include water, hydrocarbons such as hexane and heptane, aromatic hydrocarbons such as toluene, ethylbenzene and xylene, chlorinated hydrocarbons such as dichloromethane, 1,2-dichloroethane, chloroform and chlorobenzene, ethers such as anisole, tetrahydrofuran, diethyl ether, tert-butyl methyl ether, diisopropyl ether, cyclopentyl methyl ether and dibutyl ether, ketones such as acetone, ethyl methyl ketone and isobutyl methyl ketone, esters such as methyl acetate, ethyl acetate and butyl acetate, nitriles such as acetonitrile and propionitrile, and alcohols such as methanol, ethanol, isopropanol and tert-butanol. These solvents may be used alone, or two or more kinds of them may be used in combination. Among them, the reaction is carried out preferably without solvent or using ester, and further preferably ethyl acetate.

The amount of the solvent is not particularly limited, and is usually from 0.5 to 100 parts by weight, and preferably from 1 to 10 parts by weight, based on 1 part by weight of the olefin (II).

The present reaction may be carried out under normal pressure conditions, or may be carried out under pressure conditions. When pressurized, the reaction is carried out usually under the conditions of 10 MPa or less, and preferably under the conditions of 1 MPa or less, at a gauge pressure of atmospheric pressure of 0 MPa. By using the solvent described above, it is also possible to mildly and efficiently carry out the reaction under normal pressure conditions.

The present reaction is carried out usually at -80 to 100°C, and preferably at 10 to 50°C.

As the surface material of the reaction vessel and the stirring blade used in the present reaction, a material used in a general chemical reaction can be used, and an acid-resistant material is more preferable. The acid-resistant material includes HASTELLOY (registered trademark) (registered trademark of Haynes International, Inc.) such as HASTELLOY (registered trademark) A, HASTELLOY (registered trademark) B, HASTELLOY (registered trademark) C and HASTELLOY (registered trademark) D, polytetrafluoroethylene such as Teflon (registered trademark) (registered trademark of DuPont), glass, materials having equivalent performance, and the like. Here, the reaction vessel and the stirring blade may be made using these materials, or may be those where the surface of the base material made of steel plate is lined or coated with these materials.

The reaction time of the present reaction is usually 1 to 100 hours, and the end point of the reaction can be analyzed by usual analytical methods such as high performance liquid chromatography and gas chromatography, and appropriately determined.

The reaction mixture containing the ester (III) obtained by the present reaction may be used in the next reaction as it is. In addition, the ester (III) can be taken from the obtained reaction mixture by the method such as distillation, crystallization, washing and column chromatography, and may be further purified by these methods.

Examples of the ester (III) include S-(3-fluoropropyl) thioacetate, S-(3,3-difluoropropyl) thioacetate, S-(3,3,3-trifluoropropyl) thioacetate, S-(3-fluorobutyl) thioacetate, S-(4-fluorobutyl) thioacetate, S-(3,3-difluorobutyl) thioacetate, S-(3,4-difluorobutyl) thioacetate, S-(4,4-difluorobutyl) thioacetate, S-(3,3,4-trifluorobutyl) thioacetate, S-(3,4,4-trifluorobutyl) thioacetate, S-(4,4,4-trifluorobutyl) thioacetate, S-(3,3,4,4-tetrafluorobutyl) thioacetate, S-(3,4,4,4-tetrafluorobutyl) thioacetate, S-(3,3,4,4,4-pentafluorobutyl) thioacetate, S-(3-fluoropentyl) thioacetate, S-(4-fluoropentyl) thioacetate, S-(5-fluoropentyl) thioacetate, S-(3,3-difluoropentyl) thioacetate, S-(3,4-difluoropentyl) thioacetate, S-(3,5-difluoropentyl) thioacetate, S-(4,4-difluoropentyl) thioacetate, S-(4,5-difluoropentyl) thioacetate, S-(5,5-difluoropentyl) thioacetate, S-(3,3,4-trifluoropentyl) thioacetate, S-(3,3,5-trifluoropentyl) thioacetate, S-(3,4,4-trifluoropentyl) thioacetate, S-(3,4,5-trifluoropentyl) thioacetate, S-(3,5,5-trifluoropentyl) thioacetate, S-(4,4,5-trifluoropentyl) thioacetate, S-(4,5,5-trifluoropentyl) thioacetate, S-(5,5,5-trifluoropentyl) thioacetate, S-(3,3,4,4-tetrafluoropentyl) thioacetate, S-(3,3,4,5-tetrafluoropentyl) thioacetate, S-(3,3,5,5-tetrafluoropentyl) thioacetate, S-(3,4,4,5-tetrafluoropentyl) thioacetate, S-(3,4,5,5-tetrafluoropentyl) thioacetate, S-(3,5,5,5-tetrafluoropentyl) thioacetate, S-(4,4,5,5-tetrafluoropentyl) thioacetate, S-(4,5,5,5-tetrafluoropentyl) thioacetate, S-(3,3,4,4,5-pentafluoropentyl) thioacetate, S-(3,3,4,5,5-pentafluoropentyl) thioacetate, S-(3,3,5,5,5-pentafluoropentyl) thioacetate, S-(3,4,4,5,5-pentafluoropentyl) thioacetate, S-(3,4,5,5,5-pentafluoropentyl) thioacetate, S-(4,4,5,5,5-pentafluoropentyl),

S-(3,3,4,4,5,5-hexafluoropentyl) thioacetate, S-(3,3,4,5,5,5-hexafluoropentyl) thioacetate, S-(3,4,4,5,5,5-hexafluoropentyl) thioacetate, S-(3,3,4,4,5,5,5-heptafluoropentyl) thioacetate, S-(3-fluorohexyl) thioacetate, S-(4-fluorohexyl) thioacetate, S-(5-fluorohexyl) thioacetate, S-(6-fluorohexyl) thioacetate, S-(3,3-difluorohexyl) thioacetate, S-(3,4-difluorohexyl) thioacetate, S-(3,5-difluorohexyl) thioacetate, S-(3,6-difluorohexyl) thioacetate, S-(4,4-difluorohexyl) thioacetate, S-(4,5-difluorohexyl) thioacetate, S-(4,6-difluorohexyl) thioacetate, S-(5,5-difluorohexyl) thioacetate, S-(5,6-difluorohexyl) thioacetate, S-(6,6-difluorohexyl) thioacetate, S-(3,3,4-trifluorohexyl) thioacetate, S-(3,3,5-trifluorohexyl) thioacetate, S-(3,3,6-trifluorohexyl) thioacetate, S-(3,4,4-trifluorchexyl) thioacetate, S-(3,4,5-trifluorohexyl) thioacetate, S-(3,4,6-trifluorohexyl) thioacetate, S-(3,5,5-trifluorohexyl) thioacetate, S-(3,5,6-trifluorohexyl) thioacetate, S-(3,6,6-trifluorohexyl) thioacetate, S-(4,5,5-trifluorohexyl) thioacetate, S-(4,5,6-trifluorohexyl) thioacetate, S-(4,6,6-trifluorohexyl) thioacetate, S-(5,5,6-trifluorohexyl) thioacetate, S-(3,6,6-trifluorohexyl) thioacetate, S-(6,6,6-trifluorohexyl) thioacetate,

S-(3,3,4,4-tetrafluorohexyl) thioacetate, S-(3,3,4,5-tetrafluorohexyl) thioacetate, S-(3,3,4,6-tetrafluorohexyl) thioacetate, S-(3,3,5,5-tetrafluorohexyl) thioacetate, S-(3,3,5,6-tetrafluorohexyl) thioacetate, S-(3,3,6,6-tetrafluorohexyl) thioacetate, S-(3,4,4,5-tetrafluorohexyl) thioacetate, S-(3,4,4,6-tetrafluorohexyl) thioacetate, S-(3,4,5,5-tetrafluorohexyl) thioacetate, S-(3,4,5,6-tetrafluorohexyl) thioacetate, S-(3,4,6,6-tetrafluorohexyl) thioacetate, S-(3,5,5,6-tetrafluorohexyl) thioacetate, S-(3,5,6,6-tetrafluorohexyl) thioacetate, S-(3,6,6,6-tetrafluorohexyl) thioacetate, S-(4,4,5,5-tetrafluorohexyl) thioacetate, S-(4,4,5,6-tetrafluorohexyl) thioacetate, S-(4,4,6,6-tetrafluorohexyl) thioacetate, S-(4,5,5,6-tetrafluorohexyl) thioacetate, S-(4,5,6,6-tetrafluorohexyl) thioacetate, S-(4,6,6,6-tetrafluorohexyl) thioacetate, S-(5,5,6,6-tetrafluorohexyl) thioacetate, S-(5,6,6,6-tetrafluorohexyl) thioacetate, S-(3,3,4,4,5-pentafluorohexyl) thioacetate, S-(3,3,4,4,6-pentafluorohexyl) thioacetate, S-(3,3,4,5,5-pentafluorohexyl) thioacetate, S-(3,3,4,5,6-pentafluorohexyl) thioacetate, S-(3,3,4,6,6-pentafluorohexyl) thioacetate, S-(3,3,5,5,6-pentafluorohexyl) thioacetate, S-(3,3,5,6,6-pentafluorohexyl) thioacetate, S-(3,3,6,6,6-pentafluorohexyl) thioacetate, S-(3,4,4,5,5-pentafluorohexyl)-thioacetate, S-(3,4,4,5,6-pentafluorohexyl) thioacetate, S-(3,4,4,6,6-pentafluorohexyl) thioacetate, S-(3,4,5,5,6-pentafluorohexyl) thioacetate, S-(3,4,5,6,6-pentafluorohexyl) thioacetate, S-(3,4,6,6,6-pentafluorohexyl) thioacetate, S-(3,5,5,6,6-pentafluorohexyl) thioacetate, S-(3,5,6,6,6-pentafluorohexyl) thioacetate, S-(4,4,5,5,6-pentafluorohexyl) thioacetate, S-(4,4,5,6,6-pentafluorohexyl) thioacetate, S-(4,4,6,6,6-pentafluorohexyl) thioacetate, S-(4,5,5,6,6-pentafluorohexyl) thioacetate, S-(4,5,6,6,6-pentafluorohexyl) thioacetate, S-(5,5,6,6,6-pentafluorohexyl) thioacetate, S-(3,3,4,4,5,5-hexafluorohexyl) thioacetate, S-(3,3,4,4,5,6-hexafluorohexyl) thioacetate, S-(3,3,4,4,6,6-hexafluorohexyl) thioacetate, S-(3,3,4,5,5,6-hexafluorohexyl) thioacetate, S-(3,3,4,5,6,6-hexafluorohexyl) thioacetate, S-(3,3,4,6,6,6-hexafluorohexyl) thioacetate, S-(3,3,5,5,6,6-hexafluorohexyl) thioacetate, S-(3,3,5,6,6,6-hexafluorohexyl) thioacetate, S-(3,4,4,5,5,6-hexafluorohexyl) thioacetate, S-(3,4,4,5,6,6-hexafluorohexyl) thioacetate, S-(3,4,4,6,6,6-hexafluorohexyl) thioacetate, S-(3,4,5,5,6,6-hexafluorohexyl) thioacetate, S-(3,4,5,6,6,6-hexafluorohexyl) thioacetate, S-(3,5,5,6,6,6-hexafluorohexyl) thioacetate, S-(4,4,5,5,6,6-hexafluorohexyl) thioacetate, S-(4,4,5,6,6,6-hexafluorohexyl) thioacetate, S-(4,5,5,6,6,6-hexafluorohexyl) thioacetate,

S-(3,3,4,4,5,5,6-haptafluorohexyl) thioacetate, S-(3,3,4,4,5,6,6-haptafluorohexyl) thioacetate, S-(3,3,4,4,6,6,6-haptafluorohexyl) thioacetate, S-(3,3,4,5,5,6,6-haptafluorohexyl) thioacetate, S-(3,3,4,5,6,6,6-haptafluorohexyl) thioacetate, S-(3,3,5,5,6,6,6-haptafluorohexyl) thioacetate, S-(3,4,4,5,5,6,6-haptafluorohexyl) thioacetate, S-(3,4,4,5,6,6,6-haptafluorohexyl) thioacetate, S-(3,4,5,5,6,6,6-haptafluorohexyl) thioacetate, S-(4,4,5,5,6,6,6-haptafluorohexyl) thioacetate, S-(3-fluoroheptyl) thioacetate, S-(4-fluoroheptyl) thioacetate, S-(5-fluoroheptyl) thioacetate, S-(6-fluoroheptyl) thioacetate, S-(7-fluoroheptyl) thioacetate, S-(3,3-difluoroheptyl) thioacetate, S-(3,4-difluoroheptyl) thioacetate, S-(3,5-difluoroheptyl) thioacetate, S-(3,6-difluoroheptyl) thioacetate, S-(3,7-difluoroheptyl) thioacetate, S-(4,4-difluoroheptyl) thioacetate, S-(4,5-difluoroheptyl) thioacetate, S-(4,6-difluoroheptyl) thioacetate, S-(4,7-difluoroheptyl) thioacetate, S-(5,5-difluoroheptyl) thioacetate, S-(5,6-difluoroheptyl) thioacetate, S-(5,7-difluoroheptyl) thioacetate, S-(6,6-difluoroheptyl) thioacetate, S-(6,7-difluoroheptyl) thioacetate, S-(7,7-difluoroheptyl) thioacetate, S-(3,3,4-trifluoroheptyl) thioacetate, S-(3,3,5-trifluoroheptyl) thioacetate, S-(3,3,6-trifluoroheptyl) thioacetate, S-(3,3,7-trifluoroheptyl) thioacetate, S-(3,4,4-trifluoroheptyl) thioacetate, S-(3,4,5-trifluoroheptyl) thioacetate, S-(3,4,6-trifluoroheptyl) thioacetate, S-(3,4,7-trifluoroheptyl) thioacetate, S-(3,5,5-trifluoroheptyl) thioacetate, S-(3,5,6-trifluoroheptyl) thioacetate, S-(3,5,7-trifluoroheptyl) thioacetate, S-(3,6,6-trifluoroheptyl) thioacetate, S-(3,6,7-trifluoroheptyl) thioacetate, S-(3,7,7-trifluoroheptyl) thioacetate, S-(4,4,5-trifluoroheptyl) thioacetate, S-(4,4,6-trifluoroheptyl) thioacetate, S-(4,4,7-trifluoroheptyl) thioacetate, S-(4,5,5-trifluoroheptyl) thioacetate, S-(4,5,6-trifluoroheptyl) thioacetate, S-(4,5,7-trifluoroheptyl) thioacetate, S-(4,6,6-trifluoroheptyl) thioacetate, S-(4,6,7-trifluoroheptyl) thioacetate, S-(4,7,7-trifluoroheptyl) thioacetate, S-(5,5,6-trifluoroheptyl) thioacetate, S-(5,5,7-trifluoroheptyl) thioacetate, S-(5,6,6-trifluoroheptyl) thioacetate, S-(5,6,7-trifluoroheptyl) thioacetate, S-(5,7,7-trifluoroheptyl) thioacetate, S-(6,6,7-trifluoroheptyl) thioacetate, S-(6,7,7-trifluoroheptyl) thioacetate, S-(7,7,7-trifluoroheptyl) thioacetate,

S-(3,3,4,4-tetrafluoroheptyl) thioacetate, S-(3,3,4,5-tetrafluoroheptyl) thioacetate, S-(3,3,4,6-tetrafluoroheptyl) thioacetate, S-(3,3,4,7-tetrafluoroheptyl) thioacetate, S-(3,3,5,5-tetrafluoroheptyl) thioacetate, S-(3,3,5,6-tetrafluoroheptyl) thioacetate, S-(3,3,5,7-tetrafluoroheptyl) thioacetate, S-(3,3,6,6-tetrafluoroheptyl) thioacetate, S-(3,3,6,7-tetrafluoroheptyl) thioacetate, S-(3,3,7,7-tetrafluoroheptyl) thioacetate, S-(3,4,4,5-tetrafluoroheptyl) thioacetate, S-(3,4,4,6-tetrafluoroheptyl) thioacetate, S-(3,4,4,7-tetrafluoroheptyl) thioacetate, S-(3,4,5,5-tetrafluoroheptyl) thioacetate, S-(3,4,5,6-tetrafluoroheptyl) thioacetate, S-(3,4,5,7-tetrafluoroheptyl) thioacetate, S-(3,4,6,7-tetrafluoroheptyl) thioacetate, S-(3,4,7,7-tetrafluoroheptyl) thioacetate, S-(3,5,5,6-tetrafluoroheptyl) thioacetate, S-(3,5,5,7-tetrafluoroheptyl) thioacetate, S-(3,5,6,6-tetrafluoroheptyl) thioacetate, S-(3,5,6,7-tetrafluoroheptyl) thioacetate, S-(3,5,7,7-tetrafluoroheptyl) thioacetate, S-(3,6,6,7-tetrafluoroheptyl) thioacetate, S-(3,6,7,7-tetrafluoroheptyl) thioacetate, S-(3,7,7,7-tetrafluoroheptyl) thioacetate, S-(4,4,5,5-tetrafluoroheptyl) thioacetate, S-(4,4,5,6-tetrafluoroheptyl) thioacetate, S-(4,4,5,7-tetrafluoroheptyl) thioacetate, S-(4,4,6,6-tetrafluoroheptyl) thioacetate, S-(4,4,6,7-tetrafluoroheptyl) thioacetate, S-(4,4,7,7-tetrafluoroheptyl) thioacetate, S-(4,5,5,6-tetrafluoroheptyl) thioacetate, S-(4,5,5,7-tetrafluoroheptyl) thioacetate, S-(4,5,6,6-tetrafluoroheptyl) thioacetate, S-(4,5,6,7-tetrafluoroheptyl) thioacetate, S-(4,5,7,7-tetrafluoroheptyl) thioacetate, S-(4,6,6,7-tetrafluoroheptyl) thioacetate, S-(4,6,7,7-tetrafluoroheptyl) thioacetate, S-(4,7,7,7-tetrafluoroheptyl) thioacetate, S-(5,5,6,6-tetrafluoroheptyl) thioacetate, S-(5,5,6,7-tetrafluoroheptyl) thioacetate, S-(5,5,7,7-tetrafluoroheptyl) thioacetate, S-(5,6,6,7-tetrafluoroheptyl) thioacetate, S-(5,6,7,7-tetrafluoroheptyl) thioacetate, S-(5,7,7,7-tetrafluoroheptyl) thioacetate, S-(6,6,7,7-tetrafluoroheptyl) thioacetate, S-(6,7,7,7-tetrafluoroheptyl) thioacetate, S-(3,3,4,4,5-pentafluoroheptyl) thioacetate, S-(3,3,4,4,6-pentafluoroheptyl) thioacetate, S-(3,3,4,4,7-pentafluoroheptyl) thioacetate, S-(3,3,4,5,5-pentafluoroheptyl) thioacetate, S-(3,3,4,5,6-pentafluoroheptyl) thioacetate, S-(3,3,4,5,7-pentafluoroheptyl) thioacetate, S-(3,3,4,6,6-pentafluoroheptyl) thioacetate, S-(3,3,4,6,7-pentafluoroheptyl) thioacetate, S-(3,3,4,7,7-pentafluoroheptyl) thioacetate, S-(3,3,5,5,6-pentafluoroheptyl) thioacetate, S-(3,3,5,5,7-pentafluoroheptyl) thioacetate, S-(3,3,5,6,6-pentafluoroheptyl) thioacetate, S-(3,3,5,6,7-pentafluoroheptyl) thioacetate, S-(3,3,5,7,7-pentafluoroheptyl) thioacetate, S-(3,3,6,6,7-pentafluoroheptyl) thioacetate, S-(3,3,6,7,7-pentafluoroheptyl) thioacetate, S-(3,3,7,7,7-pentafluorcheptyl) thioacetate, S-(3,4,4,5,5-pentafluoroheptyl) thioacetate; S-(3,4,4,5,6-pentafluoroheptyl) thioacetate, S-(3,4,4,5,7-pentafluoroheptyl) thioacetate, S-(3,4,4,6,7-pentafluoroheptyl) thioacetate, S-(3,4,4,7,7-pentafluoroheptyl) thioacetate, S-(3,4,5,5,6-pentafluoroheptyl) thioacetate, S-(3,4,5,5,7-pentafluoroheptyl) thioacetate, S-(3,4,5,6,6-pentafluoroheptyl) thioacetate, S-(3,4,5,6,7-pentafluoroheptyl) thioacetate, S-(3,4,5,7,7-pentafluoroheptyl) thioacetate, S-(3,4,6,6,7-pentafluoroheptyl) thioacetate, S-(3,4,6,7,7-pentafluoroheptyl) thioacetate, S-(3,4,7,7,7-pentafluoroheptyl) thioacetate, S-(3,5,5,6,6-pentafluoroheptyl) thioacetate, S-(3,5,5,6,7-pentafluoroheptyl) thioacetate, S-(3,5,5,7,7-pentafluoroheptyl) thioacetate, S-(3,5,5,6,7-pentafluoroheptyl) thioacetate, S-(3,5,6,7,7-pentafluoroheptyl) thioacetate, S-(3,5,7,7,7-pentafluoroheptyl) thioacetate, S-(3,6,6,7,7-pentafluoroheptyl) thioacetate, S-(3,6,7,7,7-pentafluoroheptyl) thioacetate, S-(4,4,5,5,6-pentafluoroheptyl) thioacetate, S-(4,4,5,5,7-pentafluoroheptyl) thioacetate, S-(4,4,5,6,6-pentafluoroheptyl) thioacetate, S-(4,4,5,6,7-pentafluoroheptyl) thioacetate, S-(4,4,5,7,7-pentafluoroheptyl) thioacetate, S-(4,4,6,6,7-pentafluoroheptyl) thioacetate, S-(4,4,6,7,7-pentafluoroheptyl) thioacetate, S-(4,4,7,7,7-pentafluoroheptyl) thioacetate, S-(4,5,5,6,6-pentafluoroheptyl) thioacetate, S-(4,5,5,6,7-pentafluoroheptyl) thioacetate, S-(4,5,5,7,7-pentafluoroheptyl) thioacetate, S-(4,5,7,7,7-pentafluoroheptyl) thioacetate, S-(4,6,6,7,7-pentafluoroheptyl) thioacetate, S-(4,6,7,7,7-pentafluoroheptyl) thioacetate, S-(5,5,6,6,6-pentafluoroheptyl) thioacetate, S-(5,5,6,6,7-pentafluoroheptyl) thioacetate, S-(5,5,6,7,7-pentafluoroheptyl) thioacetate, S-(5,5,7,7,7-pentafluoroheptyl) thioacetate, S-(5,6,6,7,7-pentafluoroheptyl) thioacetate, S-(5,6,7,7,7-pentafluoroheptyl) thioacetate, S-(6,6,7,7,7-pentafluoroheptyl) thioacetate,

S-(3,3,4,4,5,5-hexafluoroheptyl) thioacetate, S-(3,3,4,4,5,6-hexafluoroheptyl) thioacetate, S-(3,3,4,4,5,7-hexafluoroheptyl) thioacetate, S-(3,3,4,4,6,6-hexafluoroheptyl) thioacetate, S-(3,3,4,4,6,7-hexafluoroheptyl) thioacetate, S-(3,3,4,4,7,7-hexafluoroheptyl) thioacetate, S-(3,3,4,5,5,6-hexafluoroheptyl) thioacetate, S-(3,3,4,5,5,7-hexafluoroheptyl) thioacetate, S-(3,3,4,5,6,6-hexafluoroheptyl) thioacetate, S-(3,3,4,5,6,7-hexafluoroheptyl) thioacetate, S-(3,3,4,5,7,7-hexafluoroheptyl) thioacetate, S-(3,3,4,6,6,7-hexafluoroheptyl) thioacetate, S-(3,3,4,6,7,7-hexafluoroheptyl) thioacetate, S-(3,3,4,7,7,7-hexafluoroheptyl) thioacetate, S-(3,3,5,5,6,6-hexafluoroheptyl) thioacetate, S-(3,3,5,5,6,7-hexafluoroheptyl) thioacetate, S-(3,3,5,5,7,7-hexafluoroheptyl) thioacetate, S-(3,3,5,6,6,7-hexafluoroheptyl) thioacetate, S-(3,3,5,6,7,7-hexafluoroheptyl) thioacetate, S-(3,3,5,7,7,7-hexafluoroheptyl) thioacetate, S-(3,3,6,6,7,7-hexafluoroheptyl) thioacetate, S-(3,3,6,7,7,7-hexafluoroheptyl) thioacetate, S-(3,4,4,5,5,6-hexafluoroheptyl) thioacetate, S-(3,4,4,5,5,7-hexafluoroheptyl) thioacetate, S-(3,4,4,5,6,6-hexafluoroheptyl) thioacetate, S-(3,4,4,5,6,7-hexafluoroheptyl) thioacetate, S-(3,4,4,5,7,7-hexafluoroheptyl) thioacetate, S-(3,4,4,6,6,7-hexafluoroheptyl) thioacetate, S-(3,4,4,6,7,7-hexafluoroheptyl) thioacetate, S-(3,4,4,7,7,7-hexafluoroheptyl) thioacetate, S-(3,4,5,5,6,6-hexafluoroheptyl) thioacetate, S-(3,4,5,5,6,7-hexafluoroheptyl) thioacetate, S-(3,4,5,5,7,7-hexafluoroheptyl) thioacetate, S-(3,4,5,6,6,7-hexafluoroheptyl) thioacetate, S-(3,4,5,6,7,7-hexafluoroheptyl) thioacetate, S-(3,4,5,7,7,7-hexafluoroheptyl) thioacetate, S-(3,4,6,6,7,7-hexafluoroheptyl) thioacetate, S-(3,4,6,7,7,7-hexafluoroheptyl) thioacetate, S-(3,5,5,6,6,7-hexafluoroheptyl) thioacetate, S-(3,5,5,6,7,7-hexafluoroheptyl) thioacetate, S-(3,5,5,7,7,7-hexafluoroheptyl) thioacetate, S-(3,5,6,6,7,7-hexafluoroheptyl) thioacetate, S-(3,5,6,7,7,7-hexafluoroheptyl) thioacetate, S-(3,6,6,7,7,7-hexafluoroheptyl) thioacetate, S-(4,4,5,5,6,6-hexafluoroheptyl) thioacetate, S-(4,4,5,5,6,7-hexafluoroheptyl) thioacetate, S-(4,4,5,5,7,7-hexafluoroheptyl) thioacetate, S-(4,4,5,6,7,7-hexafluoroheptyl) thioacetate, S-(4,4,5,7,7,7-hexafluoroheptyl) thioacetate, S-(4,4,6,6,7,7-hexafluoroheptyl) thioacetate, S-14,4, 6,7,7,7'-hexafluoroheptyl) thioacetate, S-(4,5,5,6,6,7-hexafluoroheptyl) thioacetate, S-(4,5,5,6,7,7-hexafluoroheptyl) thioacetate, S-(4,5,5,7,7,7-hexafluoroheptyl) thioacetate, S-(4,5,6,6,7,7-hexafluoroheptyl) thioacetate, S-(4,5,6,7,7,7-hexafluoroheptyl) thioacetate, S-(4,6,6,7,7,7-hexafluoroheptyl) thioacetate, S-(5,5,6,6,7,7-hexafluoroheptyl) thioacetate, S-(5,5,6,7,7,7-hexafluoroheptyl) thioacetate, S-(5,6,6,7,7,7-hexafluoroheptyl) thioacetate,

S-(3,3,4,4,5,5,6-haptafluoroheptyl) thioacetate, S-(3,3,4,4,5,5,7-haptafluoroheptyl) thioacetate, S-(3,3,4,4,5,6,6-haptafluoroheptyl) thioacetate, S-(3,3,4,4,5,6,7-haptafluoroheptyl) thioacetate, S-(3,3,4,4,5,7,7-haptafluoroheptyl) thioacetate, S-(3,3,4,4,6,6,7-haptafluoroheptyl) thioacetate, S-(3,3,4,4,6,7,7-haptafluoroheptyl) thioacetate, S-(3,3,4,4,7,7,7-haptafluoroheptyl) thioacetate, S-(3,3,4,5,5,6,6-haptafluoroheptyl) thioacetate, S-(3,3,4,5,5,6,7-haptafluoroheptyl) thioacetate, S-(3,3,4,5,5,7,7-haptafluoroheptyl) thioacetate, S-(3,3,4,5,6,6,7-haptafluoroheptyl) thioacetate, S-(3,3,4,5,6,7,7-haptafluoroheptyl) thioacetate, S-(3,3,4,5,7,7,7-haptafluoroheptyl) thioacetate, S-(3,3,4,6,6,7,7-haptafluoroheptyl) thioacetate, S-(3,3,4,6,7,7,7-haptafluoroheptyl) thioacetate, S-(3,3,5,5,6,6,7-haptafluoroheptyl) thioacetate, S-(3,3,5,5,6,7,7-haptafluoroheptyl) thioacetate, S-(3,3,5,5,7,7,7-haptafluoroheptyl) thioacetate, S-(3,3,5,6,6,7,7-haptafluoroheptyl) thioacetate, S-(3,3,5,6,7,7,7-haptafluoroheptyl) thioacetate, S-(3,3,6,6,7,7,7-haptafluoroheptyl) thioacetate, S-(3,4,4,5,5,6,6-haptafluoroheptyl) thioacetate, S-(3,4,4,5,5,6,7-haptafluoroheptyl) thioacetate, S-(3,4,4,5,5,7,7-haptafluoroheptyl) thioacetate, S-(3,4,4,5,6,6,7-haptafluoroheptyl) thioacetate, S-(3,4,4,5,6,7,7-haptafluoroheptyl) thioacetate, S-(3,4,4,5,7,7,7-haptafluoroheptyl) thioacetate, S-(3,4,4,6,6,7,7-haptafluoroheptyl) thioacetate, S-(3,4,4,6,7,7,7-haptafluoroheptyl) thioacetate, S-(3,4,5,5,6,6,7-haptafluoroheptyl) thioacetate, S-(3,4,5,5,6,7,7-haptafluoroheptyl) thioacetate, S-(3,4,5,5,7,7,7-haptafluoroheptyl) thioacetate, S-(3,4,5,6,6,7,7-haptafluoroheptyl) thioacetate, S-(3,4,5,6,7,7,7-haptafluoroheptyl) thioacetate, S-(3,4,6,6,7,7,7-haptafluoroheptyl) thioacetate, S-(3,5,5,6,6,7,7-haptafluoroheptyl) thioacetate, S-(3,5,5,6,7,7,7-haptafluoroheptyl) thioacetate, S-(3,5,6,6,7,7,7-haptafluoroheptyl) thioacetate, S-(4,4,5,5,6,6,7-haptafluoroheptyl) thioacetate, S-(4,4,5,5,6,7,7-haptafluoroheptyl) thioacetate, S-(4,4,5,5,7,7,7-haptafluoroheptyl) thioacetate, S-(4,4,5,6,6,7,7-haptafluoroheptyl) thioacetate, S-(4,4,5,6,7,7,7-haptafluoroheptyl) thioacetate, S-(4,4,6,6,7,7,7-haptafluoroheptyl) thioacetate, S-(4,5,5,6,6,7,7-haptafluoroheptyl) thioacetate, S-(4,5,5,6,7,7,7-haptafluoroheptyl) thioacetate, S-(4,5,6,6,7,7,7-haptafluoroheptyl) thioacetate, S-(5,5,6,6,7,7,7-haptafluoroheptyl) thioacetate, S-(3,3,4,4,5,5,6,6-octafluoroheptyl) thioacetate, S-(3,3,4,4,5,5,6,7-octafluoroheptyl) thioacetate, S-(3,3,4,4,5,5,7,7-octafluoroheptyl) thioacetate, S-(3,3,4,4,5,6,6,7-octafluoroheptyl) thioacetate, S-(3,3,4,4,5,6,7,7-octafluoroheptyl) thioacetate, S-(3,3,4,4,5,7,7,7-octafluoroheptyl) thioacetate, S-(3,3,4,4,6,6,7,7-octafluoroheptyl) thioacetate, S-(3,3,4,4,6,7,7,7-octafluoroheptyl) thioacetate, S-(3,3,4,5,5,6,6,7-octafluoroheptyl) thioacetate, S-(3,3,4,5,5,6,7,7-octafluoroheptyl) thioacetate,

S-(3,3,4,5,5,7,7,7-octafluoroheptyl) thioacetate, S-(3,3,4,5,6,6,7,7-octafluoroheptyl) thioacetate, S-(3,3,4,5,6,7,7,7-octafluoroheptyl) thioacetate, S-(3,3,4,6,6,7,7,7-octafluoroheptyl) thioacetate, S-(3,3,5,5,6,6,7,7-octafluoroheptyl) thioacetate, S-(3,3,5,5,6,7,7,7-octafluoroheptyl) thioacetate, S-(3,3,5,6,6,7,7,7-octafluoroheptyl) thioacetate, S-(3,4,4,5,5,6,6,7-octafluoroheptyl) thioacetate, S-(3,4,4,5,5,6,7,7-octafluoroheptyl) thioacetate, S-(3,4,4,5,5,7,7,7-octafluoroheptyl) thioacetate, S-(3,4,4,5,6,6,7,7-octafluoroheptyl) thioacetate, S-(3,4,4,5,6,7,7,7-octafluoroheptyl) thioacetate, 5-(3,4,4,6,6,7,7,7-octafluoroheptyl) thioacetate, S-(3,4,5,5,6,6,7,7-octafluoroheptyl) thioacetate, S-(3,4,5,5,5,7,7,7-octafluoroheptyl) thioacetate, S-(3,4,5,6,6,7,7,7-octafluoroheptyl) thioacetate, S-(3,5,5,6,6,7,7,7-octafluoroheptyl) thioacetate, 5-(4,4,5,5,6,6,7,7-octafluoroheptyl) thioacetate, S-(4,4,5,5,6,7,7,7-octafluoroheptyl) thioacetate, S-(4,4,5,6,6,7,7,7-octafluoroheptyl) thioacetate, S-(4,5,5,6,6,7,7,7-octafluoroheptyl) thioacetate, S-(3,3,4,4,5,5,6,6,7-nonafluoroheptyl) thioacetate, S-(3,3,4,4,5,5,6,7,7-nonafluoroheptyl) thioacetate, S-(3,3,4,4,5,5,7,7,7-nonafluoroheptyl) thioacetate, S-(3,3,4,4,5,6,6,7,7-nonafluoroheptyl) thioacetate, S-(3,3,4,4,5,6,7,7,7-nonafluoroheptyl) thioacetate, S-(3,3,4,4,6,6,7,7,7-nonafluoroheptyl) thioacetate, S-(3,3,4,5,5,6,6,7,7-nonafluoroheptyl) thioacetate, S-(3,3,4,5,5,6,7,7,7-nonafluoroheptyl) thioacetate, S-(3,3,4,5,6,6,7,7,7-nonafluoroheptyl) thioacetate, S-(3,3,5,5,6,6,7,7,7-nonafluoroheptyl) thioacetate, S-(3,4,4,5,5,6,6,7,7-nonafluoroheptyl) thioacetate, S-(3,4,4,5,5,6,7,7,7-nonafluoroheptyl) thioacetate, S-(3,4,4,5,6,6,7,7,7-nonafluoroheptyl) thioacetate, S-(3,4,5,5,6,6,7,7,7-nonafluoroheptyl) thioacetate, S-(4,4,5,5,6,6,7,7,7-nonafluoroheptyl) thioacetate, S-(3,3,4,4,5,5,6,6,7,7-decafluoroheptyl) thioacetate, S-(3,3,4,4,5,5,6,7,7,7-decafluoroheptyl) thioacetate, S-(3,3,4,4,5,6,6,7,7,7-decafluoroheptyl) thioacetate, S-(3,3,4,5,5,6,6,7,7,7-decafluoroheptyl) thioacetate, S-(3,4,4,5,5,6,6,7,7,7-decafluoroheptyl) thioacetate, S-(3,3,4,4,5,5,6,6,7,7,7-undecafluoroheptyl) thioacetate,

S-(3-fluoropropyl) thiopropionate, S-(3,3-difluoropropyl) thiopropionate, S-(3,3,3-trifluoropropyl) thiopropionate, S-(3-fluorobutyl) thiopropionate, S-(4-fluorobutyl) thiopropionate, S-(3,3-difluorobutyl) thiopropionate, S-(3,4-difluorobutyl) thiopropionate, S-(4,4-difluorobutyl) thiopropionate, S-(3,3,4-trifluorobutyl) thiopropionate, S-(3,4,4-trifluorobutyl) thiopropionate, S-(4,4,4-trifluorobutyl) thiopropionate, S-(3,3,4,4-tetrafluorobutyl) thiopropionate, S-(3,4,4,4-tetrafluorobutyl) thiopropionate, S-(3,3,4,4,4-pentafluorobutyl) thiopropionate, S-(3-fluoropentyl) thiopropionate, S-(4-fluoropentyl) thiopropionate, S-(5-fluoropentyl) thiopropionate, S-(3,3-difluoropentyl) thiopropionate, S-(3,4-difluoropentyl) thiopropionate, S-(3,5-difluoropentyl) thiopropionate, S-(4,4-difluoropentyl) thiopropionate, S-(4,5-difluoropentyl) thiopropionate, S-(5,5-difluoropentyl) thiopropionate, S-(3,3,4-trifluoropentyl) thiopropionate, S-(3,3,5-trifluoropentyl) thiopropionate, S-(3,4,4-trifluoropentyl) thiopropionate, S-(3,4,5-trifluoropentyl) thiopropionate, S-(3,5,5-trifluoropentyl) thiopropionate, S-(4,4,5-trifluoropentyl) thiopropionate, S-(4,5,5-trifluoropentyl) thiopropionate, S-(5,5,5-trifluoropentyl) thiopropionate,

S-(3,3,4,4-tetrafluoropentyl) thiopropionate, S-(3,3,4,5-tetrafluoropentyl) thiopropionate, S-(3,3,5,5-tetrafluoropentyl) thiopropionate, S-(3,4,4,5-tetrafluoropentyl) thiopropionate, S-(3,4,5,5-tetrafluoropentyl) thiopropionate, S-(3,5,5,5-tetrafluoropentyl) thiopropionate, S-(4,4,5,5-tetrafluoropentyl) thiopropionate, S-(4,5,5,5-tetrafluoropentyl) thiopropionate, S-(3,3,4,4,5-pentafluoropentyl) thiopropionate, S-(3,3,4,5,5-pentafluoropentyl) thiopropionate, S-(3,3,5,5,5-pentafluoropentyl) thiopropionate, S-(3,4,4,5,5-pentafluoropentyl) thiopropionate, S-(3,4,5,5,5-pentafluoropentyl) thiopropionate, S-(4,4,5,5,5-pentafluoropentyl) thiopropionate, S-(3,3,4,4,5,5-hexafluoropentyl) thiopropionate, S-(3,3,4,5,5,5-hexafluoropentyl) thiopropionate, S-(3,4,4,5,5,5-hexafluoropentyl) thiopropionate, S-(3,3,4,4,5,5,5-heptafluoropentyl) thiopropionate, S-(3-fluorohexyl) thiopropionate, S-(4-fluorohexyl) thiopropionate, S-(5-fluorohexyl) thiopropionate, S-(6-fluorohexyl) thiopropionate, S-(3,3-difluorohexyl) thiopropionate, S-(3,4-difluorohexyl) thiopropionate, S-(3,5-difluorohexyl) thiopropionate, S-(3,6-difluorohexyl) thiopropionate, S-(4,4-difluorohexyl) thiopropionate, S-(4,5-difluorohexyl) thiopropionate, S-(4,6-difluorohexyl) thiopropionate, S-(5,5-difluorohexyl) thiopropionate, S-(5,6-difluorohexyl) thiopropionate, S-(6,6-difluorohexyl) thiopropionate, S-(3,3,4-trifluorohexyl) thiopropionate, S-(3,3,5-trifluorohexyl) thiopropionate, S-(3,3,6-trifluorohexyl) thiopropionate, S-(3,4,4-trifluorohexyl) thiopropionate, S-(3,4,5-trifluorohexyl) thiopropionate, S-(3,4,6-trifluorohexyl) thiopropionate, S-(3,5,5-trifluorohexyl) thiopropionate, S-(3,5,6-trifluorohexyl) thiopropionate, S-(3,6,6-trifluorohexyl) thiopropionate, S-(4,5,5-trifluorohexyl) thiopropionate, S-(4,5,6-trifluorohexyl) thiopropionate, S-(4,6,6-trifluorohexyl) thiopropionate, S-(5,5,6-trifluorohexyl) thiopropionate, S-(5,6,6-trifluorohexyl) thiopropionate, S-(6,6,6-trifluorohexyl) thiopropionate, S-(3,3,4,4-tetrafluorohexyl) thiopropionate,

S-(3,3,4,5-tetrafluorohexyl) thiopropionate, S-(3,3,4,6-tetrafluorohexyl) thiopropionate, S-(3,3,5,5-tetrafluorohexyl) thiopropionate, S-(3,3,5,6-tetrafluorohexyl) thiopropionate, S-(3,3,6,6-tetrafluorohexyl) thiopropionate, S-(3,4,4,5-tetrafluorohexyl) thiopropionate, S-(3,4,4,6-tetrafluorohexyl) thiopropionate, S-(3,4,5,5-tetrafluorohexyl) thiopropionate, S-(3,4,5,6-tetrafluorohexyl) thiopropionate, S-(3,4,6,6-tetrafluorohexyl) thiopropionate, S-(3,5,5,6-tetrafluorohexyl) thiopropionate, S-(3,5,6,6-tetrafluorohexyl) thiopropionate, S-(3,6,6,6-tetrafluorohexyl) thiopropionate, S-(4,4,5,5-tetrafluorohexyl) thiopropionate, S-(4,4,5,6-tetrafluorohexyl) thiopropionate, S-(4,4,6,6-tetrafluorohexyl) thiopropionate, S-(4,5,5,6-tetrafluorohexyl) thiopropionate, S-(4,5,6,6-tetrafluorohexyl) thiopropionate, S-(4,6,6,6-tetrafluorohexyl) thiopropionate, S-(5,5,6,6-tetrafluorohexyl) thiopropionate, S-(5,6,6,6-tetrafluorohexyl) thiopropionate, S-(3,3,4,4,5-pentafluorohexyl) thiopropionate, S-(3,3,4,4,6-pentafluorohexyl) thiopropionate, S-(3,3,4,5,5-pentafluorohexyl) thiopropionate, S-(3,3,4,5,6-pentafluorohexyl) thiopropionate, S-(3,3,4,6,6-pentafluorohexyl) thiopropionate, S-(3,3,5,5,6-pentafluorohexyl) thiopropionate, S-(3,3,5,6,6-pentafluorohexyl) thiopropionate, S-(3,3,6,6,6-pentafluorohexyl) thiopropionate, S-(3,4,4,5,5-pentafluorohexyl) thiopropionate, S-(3,4,4,5,6-pentafluorohexyl) thiopropionate, S-(3,4,4,6,6-pentafluorohexyl) thiopropionate, S-(3,4,5,5,6-pentafluorohexyl) thiopropionate, S-(3,4,5,6,6-pentafluorohexyl) thiopropionate, S-(3,4,6,6,6-pentafluorohexyl) thiopropionate, S-(3,5,5,6,6-pentafluorohexyl) thiopropionate, S-(3,5,6,6,6-pentafluorohexyl) thiopropionate, S-(4,4,5,5,6-pentafluorohexyl) thiopropionate, S-(4,4,5,6,6-pentafluorohexyl) thiopropionate, S-(4,4,6,6,6-pentafluorohexyl) thiopropionate, S-(4,5,5,6,6-pentafluorohexyl) thiopropionate, S-(4,5,6,6,6-pentafluorohexyl) thiopropionate, S-(5,5,6,6,6-pentafluorohexyl) thiopropionate, S-(3,3,4,4,5,5-hexafluorohexyl) thiopropionate, S-(3,3,4,4,5,6-hexafluorohexyl) thiopropionate, S-(3,3,4,4,6,6-hexafluorohexyl) thiopropionate, S-(3,3,4,5,5,6-hexafluorohexyl) thiopropionate, S-(3,3,4,5,6,6-hexafluorohexyl) thiopropionate, S-(3,3,4,6,6,6-hexafluorohexyl) thiopropionate, S-(3,3,5,5,6,6-hexafluorohexyl) thiopropionate, S-(3,3,5,6,6,6-hexafluorohexyl) thiopropionate, S-(3,4,4,5,5,6-hexafluorohexyl) thiopropionate, S-(3,4,4,5,6,6-hexafluorohexyl) thiopropionate, S-(3,4,4,6,6,6-hexafluorohexyl) thiopropionate, S-(3,4,5,5,6,6-hexafluorohexyl) thiopropionate, S-(3,4,5,6,6,6-hexafluorohexyl) thiopropionate, S-(3,5,5,6,6,6-hexafluorohexyl) thiopropionate, S-(4,4,5,5,6,6-hexafluorohexyl) thiopropionate, S-(4,4,5,6,6,6-hexafluorohexyl) thiopropionate, S-(4,5,5,6,6,6-hexafluorohexyl) thiopropionate, S-(3,3,4,4,5,5,6-haptafluorohexyl) thiopropionate, S-(3,3,4,4,5,6,6-haptafluorohexyl) thiopropionate, S-(3,3,4,4,6,6,6-haptafluorohexyl) thiopropionate, S-(3,3,4,5,5,6,6-haptafluorohexyl) thiopropionate, S-(3,3,4,5,6,6,6-haptafluorohexyl) thiopropionate, S-(3,3,5,5,6,6,6-haptafluorohexyl) thiopropionate, S-(3,4,4,5,5,6,6-haptafluorohexyl) thiopropionate, S-(3,4,4,5,6,6,6-haptafluorohexyl) thiopropionate, S-(3,4,5,5,6,6,6-haptafluorohexyl) thiopropionate, S-(4,4,5,5,6,6,6-haptafluorohexyl) thiopropionate,

S-(3-fluoroheptyl) thiopropionate, S-(4-fluoroheptyl) thiopropionate, S-(5-fluoroheptyl) thiopropionate, S-(6-fluoroheptyl) thiopropionate, S-(7-fluoroheptyl) thiopropionate, S-(3,3-difluoroheptyl) thiopropionate, S-(3,4-difluoroheptyl) thiopropionate, S-(3,5-difluoroheptyl) thiopropionate, S-(3,6-difluoroheptyl) thiopropionate, S-(3,7-difluoroheptyl) thiopropionate, S-(4,4-difluoroheptyl) thiopropionate, S-(4,5-difluoroheptyl) thiopropionate, S-(4,6-difluoroheptyl) thiopropionate, S-(4,7-difluoroheptyl) thiopropionate, S-(5,5-difluoroheptyl) thiopropionate, S-(5,6-difluoroheptyl) thiopropionate, S-(5,7-difluoroheptyl) thiopropionate, S-(6,6-difluoroheptyl) thiopropionate, S-(6,7-difluoroheptyl) thiopropionate, S-(7,7-difluoroheptyl) thiopropionate, S-(3,3,4-trifluoroheptyl) thiopropionate, S-(3,3,5-trifluoroheptyl) thiopropionate, S-(3,3,6-trifluoroheptyl) thiopropionate, S-(3,3,7-trifluoroheptyl) thiopropionate, S-(3,4,4-trifluoroheptyl) thiopropionate, S-(3,4,5-trifluoroheptyl) thiopropionate, S-(3,4,6-trifluoroheptyl) thiopropionate, S-(3,4,7-trifluoroheptyl) thiopropionate, S-(3,5,5-trifluoroheptyl) thiopropionate, S-(3,5,6-trifluoroheptyl) thiopropionate, S-(3,5,7-trifluoroheptyl) thiopropionate, S-(3,6,6-trifluoroheptyl) thiopropionate, S-(3,6,7-trifluoroheptyl) thiopropionate, S-(3,7,7-trifluoroheptyl) thiopropionate, S-(4,4,5-trifluoroheptyl) thiopropionate, S-(4,4,6-trifluoroheptyl) thiopropionate, S-(4,4,7-trifluoroheptyl) thiopropionate, S-(4,5,5-trifluoroheptyl) thiopropionate, S-(4,5,6-trifluoroheptyl) thiopropionate, S-(4,5,7-trifluoroheptyl) thiopropionate, S-(4,6,6-trifluoroheptyl) thiopropionate, S-(4,6,7-trifluoroheptyl) thiopropionate, S-(4,7,7-trifluoroheptyl) thiopropionate, S-(5,5,6-trifluoroheptyl) thiopropionate, S-(5,5,7-trifluoroheptyl) thiopropionate, S-(5,6,6-trifluoroheptyl) thiopropionate, S-(5,6,7-trifluoroheptyl) thiopropionate, S-(5,7,7-trifluoroheptyl) thiopropionate, S-(6,6,7-trifluoroheptyl) thiopropionate, S-(6,7,7-trifluoroheptyl) thiopropionate, S-(7,7,7-trifluoroheptyl) thiopropionate,

S-(3,3,4,4-tetrafluoroheptyl) thiopropionate, S-(3,3,4,5-tetrafluoroheptyl) thiopropionate, S-(3,3,4,6-tetrafluoroheptyl) thiopropionate, S-(3,3,4,7-tetrafluoroheptyl) thiopropionate, S-(3,3,5,5-tetrafluoroheptyl) thiopropionate, S-(3,3,5,6-tetrafluoroheptyl) thiopropionate, S-(3,3,5,7-tetrafluoroheptyl) thiopropionate, S-(3,3,6,6-tetrafluoroheptyl) thiopropionate, S-(3,3,6,7-tetrafluoroheptyl) thiopropionate, S-(3,3,7,7-tetrafluoroheptyl) thiopropionate, S-(3,4,4,5-tetrafluoroheptyl) thiopropionate, S-(3,4,4,6-tetrafluoroheptyl) thiopropionate, S-(3,4,4,7-tetrafluoroheptyl) thiopropionate, S-(3,4,5,5-tetrafluoroheptyl) thiopropionate, S-(3,4,5,6-tetrafluoroheptyl) thiopropionate, S-(3,4,5,7-tetrafluoroheptyl) thiopropionate, S-(3,4,6,7-tetrafluoroheptyl) thiopropionate, S-(3,4,7,7-tetrafluoroheptyl) thiopropionate, S-(3,5,5,6-tetrafluoroheptyl) thiopropionate, S-(3,5,5,7-tetrafluoroheptyl) thiopropionate, S-(3,5,6,6-tetrafluoroheptyl) thiopropionate, S-(3,5,6,7-tetrafluoroheptyl) thiopropionate, S-(3,5,7,7-tetrafluoroheptyl) thiopropionate, S-(3,6,6,7-tetrafluoroheptyl) thiopropionate, S-(3,6,7,7-tetrafluoroheptyl) thiopropionate, S-(3,7,7,7-tetrafluoroheptyl) thiopropionate, S-(4,4,5,5-tetrafluoroheptyl) thiopropionate, S-(4,4,5,6-tetrafluoroheptyl) thiopropionate, S-(4,4,5,7-tetrafluoroheptyl) thiopropionate, S-(4,4,6,6-tetrafluoroheptyl) thiopropionate, S-(4,4,6,7-tetrafluoroheptyl) thiopropionate, S-(4,4,7,7-tetrafluoroheptyl) thiopropionate, S-(4,5,5,6-tetrafluoroheptyl) thiopropionate, S-(4,5,5,7-tetrafluoroheptyl) thiopropionate, S-(4,5,6,6-tetrafluoroheptyl) thiopropionate, S-(4,5,6,7-tetrafluoroheptyl) thiopropionate, S-(4,5,7,7-tetrafluoroheptyl) thiopropionate, S-(4,6,6,7-tetrafluoroheptyl) thiopropionate, S-(4,6,7,7-tetrafluoroheptyl) thiopropionate, S-(4,7,7,7-tetrafluoroheptyl) thiopropionate, S-(5,5,6,6-tetrafluoroheptyl) thiopropionate, S-(5,5,6,7-tetrafluoroheptyl) thiopropionate, S-(5,5,7,7-tetrafluoroheptyl) thiopropionate, S-(5,6,6,7-tetrafluoroheptyl) thiopropionate, S-(5,6,7,7-tetrafluoroheptyl) thiopropionate, S-(5,7,7,7-tetrafluoroheptyl) thiopropionate, S-(6,6,7,7-tetrafluoroheptyl) thiopropionate, S-(6,7,7,7-tetrafluoroheptyl) thiopropionate,

S-(3,3,4,4,5-pentafluoroheptyl) thiopropionate, S-(3,3,4,4,6-pentafluoroheptyl) thiopropionate, S-(3,3,4,4,7-pentafluoroheptyl) thiopropionate, S-(3,3,4,5,5-pentafluoroheptyl) thiopropionate, S-(3,3,4,5,6-pentafluoroheptyl) thiopropionate, S-(3,3,4,5,7-pentafluoroheptyl) thiopropionate, S-(3,3,4,6,6-pentafluoroheptyl) thiopropionate, S-(3,3,4,6,7-pentafluoroheptyl) thiopropionate, S-(3,3,4,7,7-pentafluoroheptyl) thiopropionate, S-(3,3,5,5,6-pentafluoroheptyl) thiopropionate, S-(3,3,5,5,7-pentafluoroheptyl) thiopropionate, S-(3,3,5,6,6-pentafluoroheptyl) thiopropionate, S-(3,3,5,6,7-pentafluoroheptyl) thiopropionate, S-(3,3,5,7,7-pentafluoroheptyl) thiopropionate, S-(3,3,6,6,7-pentafluoroheptyl) thiopropionate, S-(3,3,6,7,7-pentafluoroheptyl) thiopropionate, S-(3,3,7,7,7-pentafluoroheptyl) thiopropionate, S-(3,4,4,5,5-pentafluoroheptyl) thiopropionate, S-(3,4,4,5,6-pentafluoroheptyl) thiopropionate, S-(3,4,4,5,7-pentafluoroheptyl) thiopropionate, S-(3,4,4,6,7-pentafluoroheptyl) thiopropionate, S-(3,4,4,7,7-pentafluoroheptyl) thiopropionate, S-(3,4,5,5,6-pentafluoroheptyl) thiopropionate, S-(3,4,5,5,7-pentafluoroheptyl) thiopropionate, S-(3,4,5,6,6-pentafluoroheptyl) thiopropionate, S-(3,4,5,6,7-pentafluoroheptyl) thiopropionate, S-(3,4,5,7,7-pentafluoroheptyl) thiopropionate, S-(3,4,6,6,7-pentafluoroheptyl) thiopropionate, S-(3,4,6,7,7-pentafluoroheptyl) thiopropionate, S-(3,4,7,7,7-pentafluoroheptyl) thiopropionate, S-(3,5,5,6,6-pentafluoroheptyl) thiopropionate, S-(3,5,5,6,7-pentafluoroheptyl) thiopropionate, S-(3,5,5,7,7-pentafluoroheptyl) thiopropionate, S-(3,5,6,6,7-pentafluoroheptyl) thiopropionate, S-(3,5,6,7,7-pentafluoroheptyl) thiopropionate, S-(3,5,7,7,7-pentafluoroheptyl) thiopropionate, S-(3,6,6,7,7-pentafluoroheptyl) thiopropionate, S-(3,6,7,7,7-pentafluoroheptyl) thiopropionate, S-(4,4,5,5,6-pentafluoroheptyl) thiopropionate, S-(4,4,5,5,7-pentafluoroheptyl) thiopropionate, S-(4,4,5,6,6-pentafluoroheptyl) thiopropionate, S-(4,4,5,6,7-pentafluoroheptyl) thiopropionate, S-(4,4,5,7,7-pentafluoroheptyl) thiopropionate, S-(4,4,6,6,7-pentafluoroheptyl) thiopropionate, S-(4,4,6,7,7-pentafluoroheptyl) thiopropionate, S-(4,4,7,7,7-pentafluoroheptyl) thiopropionate, S-(4,5,5,6,6-pentafluoroheptyl) thiopropionate, S-(4,5,5,6,7-pentafluoroheptyl) thiopropionate, S-(4,5,5,7,7-pentafluoroheptyl) thiopropionate, S-(4,5,7,7,7-pentafluoroheptyl) thiopropionate, S-(4,6,6,7,7-pentafluoroheptyl) thiopropionate, S-(4,6,7,7,7-pentafluoroheptyl) thiopropionate, S-(5,5,6,6,6-pentafluoroheptyl) thiopropionate, S-(5,5,6,6,7-pentafluoroheptyl) thiopropionate, S-(5,5,6,7,7-pentafluoroheptyl) thiopropionate, S-(5,5,7,7,7-pentafluoroheptyl) thiopropionate, S-(5,6,6,7,7-pentafluoroheptyl) thiopropionate, S-(5,6,7,7,7-pentafluoroheptyl) thiopropionate, S-(6,6,7,7,7-pentafluoroheptyl) thiopropionate,

S-(3,3,4,4,5,5-hexafluoroheptyl) thiopropionate, S-(3,3,4,4,5,6-hexafluoroheptyl) thiopropionate, S-(3,3,4,4,5,7-hexafluoroheptyl) thiopropionate, S-(3,3,4,4,6,6-hexafluoroheptyl) thiopropionate, S-(3,3,4,4,6,7-hexafluoroheptyl) thiopropionate, S-(3,3,4,4,7,7-hexafluoroheptyl) thiopropionate, S-(3,3,4,5,5,6-hexafluoroheptyl) thiopropionate, S-(3,3,4,5,5,7-hexafluoroheptyl) thiopropionate, S-(3,3,4,5,6,6-hexafluoroheptyl) thiopropionate, S-(3,3,4,5,6,7-hexafluoroheptyl) thiopropionate, S-(3,3,4,5,7,7-hexafluoroheptyl) thiopropionate, S-(3,3,4,6,6,7-hexafluoroheptyl) thiopropionate, S-(3,3,4,6,7,7-hexafluoroheptyl) thiopropionate, S-(3,3,4,7,7,7-hexafluoroheptyl) thiopropionate, S-(3,3,5,5,6,6-hexafluoroheptyl) thiopropionate, S-(3,3,5,5,6,7-hexafluoroheptyl) thiopropionate, S-(3,3,5,5,7,7-hexafluoroheptyl) thiopropionate, S-(3,3,5,6,6,7-hexafluoroheptyl) thiopropionate, S-(3,3,5,6,7,7-hexafluoroheptyl) thiopropionate, S-(3,3,5,7,7,7-hexafluoroheptyl) thiopropionate, S-(3,3,6,6,7,7-hexafluoroheptyl) thiopropionate, S-(3,3,6,7,7,7-hexafluoroheptyl) thiopropionate, S-(3,4,4,5,5,6-hexafluoroheptyl) thiopropionate, S-(3,4,4,5,5,7-hexafluoroheptyl) thiopropionate, S-(3,4,4,5,6,6-hexafluoroheptyl) thiopropionate, S-(3,4,4,5,6,7-hexafluoroheptyl) thiopropionate, S-(3,4,4,5,7,7-hexafluoroheptyl) thiopropionate, S-(3,4,4,6,6,7-hexafluoroheptyl) thiopropionate, S-(3,4,4,6,7,7-hexafluoroheptyl) thiopropionate, S-(3,4,4,7,7,7-hexafluoroheptyl) thiopropionate, S-(3,4,5,5,6,6-hexafluoroheptyl) thioproplonate, S-(3,4,5,5,6,7-hexafluoroheptyl) thiopropionate, S-(3,4,5,5,7,7-hexafluoroheptyl) thiopropionate, S-(3,4,5,6,6,7-hexafluoroheptyl) thiopropionate, S-(3,4,5,6,7,7-hexafluoroheptyl) thiopropionate, S-(3,4,5,7,7,7-hexafluoroheptyl) thiopropionate, S-(3,4,6,6,7,7-hexafluoroheptyl) thiopropionate, S-(3,4,6,7,7,7-hexafluoroheptyl) thiopropionate, S-(3,5,5,6,6,7-hexafluoroheptyl) thiopropionate, S-(3,5,5,6,7,7-hexafluoroheptyl) thiopropionate, S-(3,5,5,7,7,7-hexafluoroheptyl) thiopropionate, S-(3,5,6,6,7,7-hexafluoroheptyl) thiopropionate, S-(3,5,6,7,7,7-hexafluoroheptyl) thiopropionate, S-(3,6,6,7,7,7-hexafluoroheptyl) thiopropionate, S-(4,4,5,5,6,6-hexafluoroheptyl) thiopropionate, S-(4,4,5,5,6,7-hexafluoroheptyl) thiopropionate, S-(4,4,5,5,7,7-hexafluoroheptyl) thiopropionate, S-(4,4,5,6,7,7-hexafluoroheptyl) thiopropionate, S-(4,4,5,7,7,7-hexafluoroheptyl) thiopropionate, S-(4,4,6,6,7,7-hexafluoroheptyl) thiopropionate, S-(4,4,6,7,7,7-hexafluoroheptyl) thiopropionate, S-(4,5,5,6,6,7-hexafluoroheptyl) thiopropionate, S-(4,5,5,6,7,7-hexafluoroheptyl) thiopropionate, S-(4,5,5,7,7,7-hexafluoroheptyl) thiopropionate, S-(4,5,6,6,7,7-hexafluoroheptyl) thiopropionate, S-(4,5,6,7,7,7-hexafluoroheptyl) thiopropionate, S-(4,6,6,7,7,7-hexafluoroheptyl) thiopropionate, S-(5,5,6,6,7,7-hexafluoroheptyl) thiopropionate, S-(5,5,6,7,7,7-hexafluoroheptyl) thiopropionate, S-(5,6,6,7,7,7-hexafluoroheptyl) thiopropionate,

S-(3,3,4,4,5,5,6-haptafluoroheptyl) thiopropionate, S-(3,3,4,4,5,5,7-haptafluoroheptyl) thiopropionate, S-(3,3,4,4,5,6,6-haptafluoroheptyl) thiopropionate, S-(3,3,4,4,5,6,7-haptafluoroheptyl) thiopropionate, S-(3,3,4,4,5,7,7-haptafluoroheptyl) thiopropionate, S-(3,3,4,4,6,6,7-haptafluoroheptyl) thiopropionate, S-(3,3,4,4,6,7,7-haptafluoroheptyl) thiopropionate, S-(3,3,4,4,7,7,7-haptafluoroheptyl) thiopropionate, 5-(3,3,4,5,5,6,6-haptafluoroheptyl) thiopropionate, S-(3,3,4,5,5,6,7-haptafluoroheptyl) thiopropionate, S-(3,3,4,5,5,7,7-haptafluoroheptyl) thiopropionate, S-(3,3,4,5,6,6,7-haptafluoroheptyl) thiopropionate, S-(3,3,4,5,6,7,7-haptafluoroheptyl) thiopropionate, S-(3,3,4,5,7,7,7-haptafluoroheptyl) thiopropionate, S-(3,3,4,6,6,7,7-haptafluoroheptyl) thiopropionate, S-(3,3,4,6,7,7,7-haptafluoroheptyl) thiopropionate, S-(3,3,5,5,6,6,7-haptafluoroheptyl) thiopropionate, S-(3,3,5,5,6,7,7-haptafluoroheptyl) thiopropionate, S-(3,3,5,5,7,7,7-haptafluoroheptyl) thiopropionate, S-(3,3,5,6,6,7,7-haptafluoroheptyl) thiopropionate, S-(3,3,5,6,7,7,7-haptafluoroheptyl) thiopropionate, S-(3,3,6,6,7,7,7-haptafluoroheptyl) thiopropionate, S-(3,4,4,5,5,6,6-haptafluoroheptyl) thiopropionate, S-(3,4,4,5,5,6,7-haptafluoroheptyl) thiopropionate, S-(3,4,4,5,5,7,7-haptafluoroheptyl) thiopropionate, S-(3,4,4,5,6,6,7-haptafluoroheptyl) thiopropionate, S-(3,4,4,5,6,7,7-haptafluoroheptyl) thiopropionate, S-(3,4,4,5,7,7,7-haptafluoroheptyl) thiopropionate, S-(3,4,4,6,6,7,7-haptafluoroheptyl) thiopropionate, S-(3,4,4,6,7,7,7-haptafluoroheptyl) thiopropionate, S-(3,4,5,5,6,6,7-haptafluoroheptyl) thiopropionate, S-(3,4,5,5,6,7,7-haptafluoroheptyl) thiopropionate, S-(3,4,5,5,7,7,7-haptafluoroheptyl) thiopropionate, S-(3,4,5,6,6,7,7-haptafluoroheptyl) thiopropionate, S-(3,4,5,6,7,7,7-haptafluoroheptyl) thiopropionate, S-(3,4,6,6,7,7,7-haptafluoroheptyl) thiopropionate, S-(3,5,5,6,6,7,7-haptafluoroheptyl) thiopropionate, S-(3,5,5,6,7,7,7-haptafluoroheptyl) thiopropionate, S-(3,5,6,6,7,7,7-haptafluoroheptyl) thiopropionate, S-(4,4,5,5,6,6,7-haptafluoroheptyl) thiopropionate, S-(4,4,5,5,6,7,7-haptafluoroheptyl) thiopropionate, S-(4,4,5,5,7,7,7-haptafluoroheptyl) thiopropionate, S-(4,4,5,6,6,7,7-haptafluoroheptyl) thiopropionate, S-(4,4,5,6,7,7,7-haptafluoroheptyl) thiopropionate, S-(4,4,6,6,7,7,7-haptafluoroheptyl) thiopropionate, S-(4,5,5,6,6,7,7-haptafluoroheptyl) thiopropionate, S-(4,5,5,6,7,7,7-haptafluoroheptyl) thiopropionate, S-(4,5,6,6,7,7,7-haptafluoroheptyl) thiopropionate, S-(5,5,6,6,7,7,7-haptafluoroheptyl) thiopropionate,

S-(3,3,4,4,5,5,6,6-octafluoroheptyl) thiopropionate, S-(3,3,4,4,5,5,6,7-octafluoroheptyl) thiopropionate, S-(3,3,4,4,5,5,7,7-octafluoroheptyl) thiopropionate, S-(3,3,4,4,5,6,6,7-octafluoroheptyl) thiopropionate, S-(3,3,4,4,5,6,7,7-octafluoroheptyl) thiopropionate, S-(3,3,4,4,5,7,7,7-octafluoroheptyl) thiopropionate, S-(3,3,4,4,6,6,7,7-octafluoroheptyl) thiopropionate, S-(3,3,4,4,6,7,7,7-octafluoroheptyl) thiopropionate, S-(3,3,4,5,5,6,6,7-octafluoroheptyl) thiopropionate, S-(3,3,4,5,5,6,7,7-octafluoroheptyl) thiopropionate, S-(3,3,4,5,5,7,7,7-octafluoroheptyl) thiopropionate, S-(3,3,4,5,6,6,7,7-octafluoroheptyl) thiopropionate, S-(3,3,4,5,6,7,7,7-octafluoroheptyl) thiopropionate, S-(3,3,4,6,6,7,7,7-octafluoroheptyl) thiopropionate, S-(3,3,5,5,6,6,7,7-octafluoroheptyl) thiopropionate, S-(3,3,5,5,6,7,7,7-octafluoroheptyl) thiopropionate, S-(3,3,5,6,6,7,7,7-octafluoroheptyl) thiopropionate, S-(3,4,4,5,5,6,6,7-octafluoroheptyl) thiopropionate, S-(3,4,4,5,5,6,7,7-octafluoroheptyl) thiopropionate, S-(3,4,4,5,5,7,7,7-octafluoroheptyl) thiopropionate, S-(3,4,4,5,6,6,7,7-octafluoroheptyl) thiopropionate, S-(3,4,4,5,6,7,7,7-octafluoroheptyl) thiopropionate, S-(3,4,4,6,6,7,7,7-octafluoroheptyl) thiopropionate, S-(3,4,5,5,6,6,7,7-octafluoroheptyl) thiopropionate, S-(3,4,5,5,6,7,7,7-octafluoroheptyl) thiopropionate, S-(3,4,5,6,6,7,7,7-octafluoroheptyl) thiopropionate, S-(3,5,5,6,6,7,7,7-octafluoroheptyl) thiopropionate, S-(4,4,5,5,6,6,7,7-octafluoroheptyl) thiopropionate, S-(4,4,5,5,6,7,7,7-octafluoroheptyl) thiopropionate, S-(4,4,5,6,6,7,7,7-octafluoroheptyl) thiopropionate, S-(4,5,5,6,6,7,7,7-octafluoroheptyl) thiopropionate,

S-(3,3,4,4,5,5,6,6,7-nonafluoroheptyl) thiopropionate, S-(3,3,4,4,5,5,6,7,7-nonafluoroheptyl) thiopropionate, S-(3,3,4,4,5,5,7,7,7-nonafluoroheptyl) thiopropionate, S-(3,3;4,4,5,6,6,7,7-nonafluoroheptyl) thiopropionate, S-(3,3,4,4,5,6,7,7,7-nonafluoroheptyl) thiopropionate, S-(3,3,4,4,6,6,7,7,7-nonafluoroheptyl) thiopropionate, S-(3,3,4,5,5,6,6,7,7-nonafluoroheptyl) thiopropionate, S-(3,3,4,5,5,6,7,7,7-nonafluoroheptyl) thiopropionate, S-(3,3,4,5,6,6,7,7,7-nonafluoroheptyl) thiopropionate, S-(3,3,5,5,6,6,7,7,7-nonafluoroheptyl) thiopropionate, S-(3,4,4,5,5,6,6,7,7-nonafluoroheptyl) thiopropionate, S-(3,4,4,5,5,6,7,7,7-nonafluoroheptyl) thiopropionate, S-(3,4,4,5,6,6,7,7,7-nonafluoroheptyl) thiopropionate, S-(3,4,5,5,6,6,7,7,7-nonafluoroheptyl) thiopropionate, S-(4,4,5,5,6,6,7,7,7-nonafluoroheptyl) thiopropionate, S-(3,3,4,4,5,5,6,6,7,7-decafluoroheptyl) thiopropionate, S-(3,3,4,4,5,5,6,7,7,7-decafluoroheptyl) thiopropionate, S-(3,3,4,4,5,6,6,7,7,7-decafluoroheptyl) thiopropionate, S-(3,3,4,5,5,6,6,7,7,7-decafluoroheptyl) thiopropionate, S-(3,4,4,5,5,6,6,7,7,7-decafluoroheptyl) thiopropionate, S-(3,3,4,4,5,5,6,6,7,7,7-undecafluoroheptyl) thiopropionate,

S-(3-fluoropropyl) thiobenzoate, S-(3,3-difluoropropyl) thiobenzoate, S-(3,3,3-trifluoropropyl) thiobenzoate, S-(3-fluorobutyl) thiobenzoate, S-(4-fluorobutyl) thiobenzoate, S-(3,3-difluorobutyl) thiobenzoate, S-(3,4-difluorobutyl) thiobenzoate, S-(4,4-difluorobutyl) thiobenzoate, S-(3,3,4-trifluorobutyl) thiobenzoate, S-(3,4,4-trifluorobutyl) thiobenzoate, S-(4,4,4-trifluorobutyl) thiobenzoate, S-(3,3;4,4-tetrafluorobutyl) thiobenzoate, S-(3,4,4,4-tetrafluorobutyl) thiobenzoate, S-(3,3,4,4,4-pentafluorobutyl) thiobenzoate, S-(3-fluoropentyl) thiobenzoate, S-(4-fluoropentyl) thiobenzoate, S-(5-fluoropentyl) thiobenzoate, S-(3,3-difluoropentyl) thiobenzoate, S-(3,4-difluoropentyl) thiobenzoate, S-(3,5-difluoropentyl) thiobenzoate, S-(4,4-difluoropentyl) thiobenzoate, S-(4,5-difluoropentyl) thiobenzoate, S-(5,5-difluoropentyl) thiobenzoate, S-(3,3,4-trifluoropentyl) thiobenzoate, S-(3,3,5-triflucropentyl) thiobenzoate, S-(3,4,4-trifluoropentyl) thiobenzoate, S-(3,4,5-trifluoropentyl) thiobenzoate, S-(3,5,5-trifluoropentyl) thiobenzoate, S-(4,4,5-trifluoropentyl) thiobenzoate, S-(4,5,5-trifluoropentyl) thiobenzoate, S-(5,5,5-trifluoropentyl) thiobenzoate, S-(3,3,4,4-tetrafluoropentyl) thiobenzoate, S-(3,3,4,5-tetrafluoropentyl) thiobenzoate, S-(3,3,5,5-tetrafluoropentyl) thiobenzoate, S-(3,4,4,5-tetrafluoropentyl) thiobenzoate, S-(3,4,5,5-tetrafluoropentyl) thiobenzoate, S-(3,5,5,5-tetrafluoropentyl) thiobenzoate, S-(4,4,5,5-tetrafluoropentyl) thiobenzoate, S-(4,5,5,5-tetrafluoropentyl) thiobenzoate, S-(3,3,4,4,5-pentafluoropentyl) thiobenzoate, S-(3,3,4,5,5-pentafluoropentyl) thiobenzoate, S-(3,3,5,5,5-pentafluoropentyl) thiobenzoate, S-(3,4,4,5,5-pentafluoropentyl) thiobenzoate, S-(3,4,5,5,5-pentafluoropentyl) thiobenzoate, S-(4,4,5,5,5-pentafluoropentyl) thiobenzoate, S-(3,3,4,4,5,5-hexafluoropentyl) thiobenzoate, S-(3,3,4,5,5,5-hexafluoropentyl) thiobenzoate, S-(3,4,4,5,5,5-hexafluoropentyl) thiobenzoate, S-(3,3,4,4,5,5,5-heptafluoropentyl) thiobenzoate, S-(3-fluorohexyl) thiobenzoate, S-(4-fluorohexyl) thiobenzoate, S-(5-fluorohexyl) thiobenzoate, S-(6-fluorohexyl) thiobenzoate, S-(3,3-difluorohexyl) thiobenzoate, S-(3,4-difluorohexyl) thiobenzoate, S-(3,5-difluorohexyl) thiobenzoate, S-(3,6-difluorohexyl) thiobenzoate, S-(4,4-difluorohexyl) thiobenzoate, S-(4,5-difluorohexyl) thiobenzoate, S-(4,6-difluorohexyl) thiobenzoate, S-(5,5-difluorohexyl) thiobenzoate, S-(5,6-difluorohexyl) thiobenzoate, S-(6,6-difluorohexyl) thiobenzoate,

S-(3,3,4-trifluorohexyl) thiobenzoate, S-(3,3,5-trifluorohexyl) thiobenzoate, S-(3,3,6-trifluorohexyl) thiobenzoate, S-(3,4,4-trifluorohexyl) thiobenzoate, S-(3,4,5-trifluorohexyl) thiobenzoate, S-(3,4,6-trifluorohexyl) thiobenzoate, S-(3,5,5-trifluorohexyl) thiobenzoate, S-(3,5,6-trifluorohexyl) thiobenzoate, S-(3,6,6-trifluorohexyl) thiobenzoate, S-(4,5,5-trifluorohexyl) thiobenzoate, S-(4,5,6-trifluorohexyl) thiobenzoate, S-(4,6,6-trifluorohexyl) thiobenzoate, S-(5,5,6-trifluorohexyl) thiobenzoate, S-(5,6,6-trifluorohexyl) thiobenzoate, S-(6,6,6-trifluorohexyl) thiobenzoate, S-(3,3,4,4-tetrafluorohexyl) thiobenzoate, S-(3,3,4,5-tetrafluorohexyl) thiobenzoate, S-(3,3,4,6-tetrafluorohexyl) thiobenzoate, S-(3,3,5,5-tetrafluorohexyl) thiobenzoate, S-(3,3,5,6-tetrafluorohexyl) thiobenzoate, S-(3,3,6,6-tetrafluorohexyl) thiobenzoate, S-(3,4,4,5-tetrafluorohexyl) thiobenzoate, S-(3,4,4,6-tetrafluorohexyl) thiobenzoate, S-(3,4,5,5-tetrafluorohexyl) thiobenzoate, S-(3,4,5,6-tetrafluorohexyl) thiobenzoate, S-(3,4,6,6-tetrafluorohexyl) thiobenzoate, S-(3,5,5,6-tetrafluorohexyl) thiobenzoate, S-(3,5,6,6-tetrafluorohexyl) thiobenzoate, S-(3,6,6,6-tetrafluorohexyl) thiobenzoate, S-(4,4,5,5-tetrafluorohexyl) thiobenzoate, S-(4,4,5,6-tetrafluorohexyl) thiobenzoate, S-(4,4,6,6-tetrafluorohexyl) thiobenzoate, S-(4,5,5,6-tetrafluorohexyl) thiobenzoate, S-(4,5,6,6-tetrafluorohexyl) thiobenzoate, S-(4,6,6,6-tetrafluorohexyl) thiobenzoate, S-(5,5,6,6-tetrafluorohexyl) thiobenzoate, S-(5,6,6,6-tetrafluorohexyl) thiobenzoate, S-(3,3,4,4,5-pentafluorohexyl) thiobenzoate, S-(3,3,4,4,6-pentafluorohexyl) thiobenzoate, S-(3,3,4,5,5-pentafluorohexyl) thiobenzoate, S-(3,3,4,5,6-pentafluorohexyl) thiobenzoate, S-(3,3,4,6,6-pentafluorohexyl) thiobenzoate, S-(3,3,5,5,6-pentafluorohexyl) thiobenzoate, S-(3,3,5,6,6-pentafluorohexyl) thiobenzoate, S-(3,3,6,6,6-pentafluorohexyl) thiobenzoate, S-(3,4,4,5,5-pentafluorohexyl) thiobenzoate, S-(3,4,4,5,6-pentafluorohexyl) thiobenzoate, S-(3,4,4,6,6-pentafluorohexyl) thiobenzoate, S-(3,4,5,5,6-pentafluorohexyl) thiobenzoate, S-(3,4,5,6,6-pentafluorohexyl) thiobenzoate, S-(3,4,6,6,6-pentafluorohexyl) thiobenzoate, S-(3,5,5,6,6-pentafluorohexyl) thiobenzoate, S-(3,5,6,6,6-pentafluorohexyl) thiobenzoate, S-(4,4,5,5,6-pentafluorohexyl) thiobenzoate, S-(4,4,5,6,6-pentafluorohexyl) thiobenzoate, S-(4,4,6,6,6-pentafluorohexyl) thiobenzoate, S-(4,5,5,6,6-pentafluorohexyl) thiobenzoate, S-(4,5,6,6,6-pentafluorohexyl) thiobenzoate, S-(5,5,6,6,6-pentafluorohexyl) thiobenzoate,

S-(3,3,4,4,5,5-hexafluorohexyl) thiobenzoate, S-(3,3,4,4,5,6-hexafluorohexyl) thiobenzoate, S-(3,3,4,4,6,6-hexafluorohexyl) thiobenzoate, S-(3,3,4,5,5,6-hexafluorohexyl) thiobenzoate, S-(3,3,4,5,6,6-hexafluorohexyl) thiobenzoate, S-(3,3,4,6,6,6-hexafluorohexyl) thiobenzoate, S-(3,3,5,5,6,6-hexafluorohexyl) thiobenzoate, S-(3,3,5,6,6,6-hexafluorohexyl) thiobenzoate, S-(3,4,4,5,5,6-hexafluorohexyl) thiobenzoate, S-(3,4,4,5,6,6-hexafluorohexyl) thiobenzoate, S-(3,4,4,6,6,6-hexafluorohexyl) thiobenzoate, S-(3,4,5,5,6,6-hexafluorohexyl) thiobenzoate, S-(3,4,5,6,6,6-hexafluorohexyl) thiobenzoate, S-(3,5,5,6,6,6-hexafluorohexyl) thiobenzoate, S-(4,4,5,5,6,6-hexafluorohexyl) thiobenzoate, S-(4,4,5,6,6,6-hexafluorohexyl) thiobenzoate, S-(4,5,5,6,6,6-hexafluorohexyl) thiobenzoate, S-(3,3,4,4,5,5,6-haptafluorohexyl) thiobenzoate, S-(3,3,4,4,5,6,6-haptafluorohexyl) thiobenzoate, S-(3,3,4,4,6,6,6-haptafluorohexyl) thiobenzoate, S-(3,3,4,5,5,6,6-haptafluorohexyl) thiobenzoate, S-(3,3,4,5,6,6,6-haptafluorohexyl) thiobenzoate, S-(3,3,5,5,6,6,6-haptafluorohexyl) thiobenzoate, S-(3,4,4,5,5,6,6-haptafluorohexyl) thiobenzoate, S-(3,4,4,5,6,6,6-haptafluorohexyl) thiobenzoate, S-(3,4,5,5,6,6,6-haptafluorohexyl) thiobenzoate, S-(4,4,5,5,6,6,6-haptafluorohexyl) thiobenzoate,

S-(3-fluoroheptyl) thiobenzoate, S-(4-fluoroheptyl) thiobenzoate, S-(5-fluoroheptyl) thiobenzoate, S-(6-fluoroheptyl) thiobenzoate, S-(7-fluoroheptyl) thiobenzoate, S-(3,3-difluoroheptyl) thiobenzoate, S-(3,4-difluoroheptyl) thiobenzoate, S-(3,5-difluoroheptyl) thiobenzoate, S-(3,6-difluoroheptyl) thiobenzoate, S-(3,7-difluoroheptyl) thiobenzoate, S-(4,4-difluoroheptyl) thiobenzoate, S-(4,5-difluoroheptyl) thiobenzoate, S-(4,6-difluoroheptyl) thiobenzoate, S-(4,7-difluoroheptyl) thiobenzoate, S-(5,5-difluoroheptyl) thiobenzoate, S-(5,6-difluoroheptyl) thiobenzoate, S-(5,7-difluoroheptyl) thiobenzoate, S-(6,6-difluoroheptyl) thiobenzoate, S-(6,7-difluoroheptyl) thiobenzoate, S-(7,7-difluoroheptyl) thiobenzoate, S-(3,3,4-trifluoroheptyl) thiobenzoate, S-(3,3,5-trifluoroheptyl) thiobenzoate, S-(3,3,6-trifluoroheptyl) thiobenzoate, S-(3,3,7-trifluoroheptyl) thiobenzoate, S-(3,4,4-trifluoroheptyl) thiobenzoate, S-(3,4,5-trifluoroheptyl) thiobenzoate, S-(3,4,6-trifluoroheptyl) thiobenzoate, S-(3,4,7-trifluoroheptyl) thiobenzoate, S-(3,5,5-trifluoroheptyl) thiobenzoate, S-(3,5,6-trifluoroheptyl) thiobenzoate, S-(3,5,7-trifluoroheptyl) thiobenzoate, S-(3,6,6-trifluoroheptyl) thiobenzoate, S-(3,6,7-trifluoroheptyl) thiobenzoate, S-(3,7,7-trifluoroheptyl) thiobenzoate, S-(4,4,5-trifluoroheptyl) thiobenzoate, S-(4,4,6-trifluoroheptyl) thiobenzoate, S-(4,4,7-trifluoroheptyl) thiobenzoate, S-(4,5,5-trifluoroheptyl) thiobenzoate, S-(4,5,6-trifluoroheptyl) thiobenzoate, S-(4,5,7-trifluoroheptyl) thiobenzoate, S-(4,6,6-trifluoroheptyl) thiobenzoate, S-(4,6,7-trifluoroheptyl) thiobenzoate, S-(4,7,7-trifluoroheptyl) thiobenzoate, S-(5,5,6-trifluoroheptyl) thiobenzoate, S-(5,5,7-trifluoroheptyl) thiobenzoate, S-(5,6,6-trifluoroheptyl) thiobenzoate, S-(5,6,7-trifluoroheptyl) thiobenzoate, S-(5,7,7-trifluoroheptyl) thiobenzoate, S-(6,6,7-trifluoroheptyl) thiobenzoate, S-(6,7,7-trifluoroheptyl) thiobenzoate, S-(7,7,7-trifluoroheptyl) thiobenzoate,

S-(3,3,4,4-tetrafluoroheptyl) thiobenzoate, S-(3,3,4,5-tetrafluoroheptyl) thiobenzoate, S-(3,3,4,6-tetrafluoroheptyl) thiobenzoate, S-(3,3,4,7-tetrafluoroheptyl) thiobenzoate, S-(3,3,5,5-tetrafluoroheptyl) thiobenzoate, S-(3,3,5,6-tetrafluoroheptyl) thiobenzoate, S-(3,3,5,7-tetrafluoroheptyl) thiobenzoate, S-(3,3,6,6-tetrafluoroheptyl) thiobenzoate, S-(3,3,6,7-tetrafluoroheptyl) thiobenzoate, S-(3,3,7,7-tetrafluoroheptyl) thiobenzoate, S-(3,4,4,5-tetrafluoroheptyl) thiobenzoate, S-(3,4,4,6-tetrafluoroheptyl) thiobenzoate, S-(3,4,4,7-tetrafluoroheptyl) thiobenzoate, S-(3,4,5,5-tetrafluoroheptyl) thiobenzoate, S-(3,4,5,6-tetrafluoroheptyl) thiobenzoate, S-(3,4,5,7-tetrafluoroheptyl) thiobenzoate, S-(3,4,6,7-tetrafluoroheptyl) thiobenzoate, S-(3,4,7,7-tetrafluoroheptyl) thiobenzoate, S-(3,5,5,6-tetrafluoroheptyl) thiobenzoate, S-(3,5,5,7-tetrafluoroheptyl) thiobenzoate, S-(3,5,6,6-tetrafluoroheptyl) thiobenzoate, S-(3,5,6,7-tetrafluoroheptyl) thiobenzoate, S-(3,5,7,7-tetrafluoroheptyl) thiobenzoate, S-(3,6,6,7-tetrafluoroheptyl) thiobenzoate, S-(3,6,7,7-tetrafluoroheptyl) thiobenzoate, S-(3,7,7,7-tetrafluoroheptyl) thiobenzoate, S-(4,4,5,5-tetrafluoroheptyl) thiobenzoate, S-(4,4,5,6-tetrafluoroheptyl) thiobenzoate, S-(4,4,5,7-tetrafluoroheptyl) thiobenzoate, S-(4,4,6,6-tetrafluoroheptyl) thiobenzoate, S-(4,4,6,7-tetrafluoroheptyl) thiobenzoate, S-(4,4,7,7-tetrafluoroheptyl) thiobenzoate, S-(4,5,5,6-tetrafluoroheptyl) thiobenzoate, S-(4,5,5,7-tetrafluoroheptyl) thiobenzoate, S-(4,5,6,6-tetrafluoroheptyl) thiobenzoate, S-(4,5,6,7-tetrafluoroheptyl) thiobenzoate, S-(4,5,7,7-tetrafluoroheptyl) thiobenzoate, S-(4,6,6,7-tetrafluoroheptyl) thiobenzoate, S-(4,6,7,7-tetrafluoroheptyl) thiobenzoate, S-(4,7,7,7-tetrafluoroheptyl) thiobenzoate, S-(5,5,6,6-tetrafluoroheptyl) thiobenzoate, S-(5,5,6,7-tetrafluoroheptyl) thiobenzoate, S-(5,5,7,7-tetrafluoroheptyl) thiobenzoate, S-(5,6,6,7-tetrafluoroheptyl) thiobenzoate, S-(5,6,7,7-tetrafluoroheptyl) thiobenzoate, S-(5,7,7,7-tetrafluoroheptyl) thiobenzoate, S-(6,6,7,7-tetrafluoroheptyl) thiobenzoate, S-(6,7,7,7-tetrafluoroheptyl) thiobenzoate,

S-(3,3,4,4,5-pentafluoroheptyl) thiobenzoate, S-(3,3,4,4,6-pentafluoroheptyl) thiobenzoate, S-(3,3,4,4,7-pentafluoroheptyl) thiobenzoate, S-(3,3,4,5,5-pentafluoroheptyl) thiobenzoate, S-(3,3,4,5,6-pentafluoroheptyl) thiobenzoate, S-(3,3,4,5,7-pentafluoroheptyl) thiobenzoate, S-(3,3,4,6,6-pentafluoroheptyl) thiobenzoate, S-(3,3,4,6,7-pentafluoroheptyl) thiobenzoate, S-(3,3,4,7,7-pentafluoroheptyl) thiobenzoate, S-(3,3,5,5,6-pentafluoroheptyl) thiobenzoate, S-(3,3,5,5,7-pentafluoroheptyl) thiobenzoate, S-(3,3,5,6,6-pentafluoroheptyl) thiobenzoate, S-(3,3,5,6,7-pentafluoroheptyl) thiobenzoate, S-(3,3,5,7,7-pentafluoroheptyl) thiobenzoate, S-(3,3,6,6,7-pentafluoroheptyl) thiobenzoate, S-(3,3,6,7,7-pentafluoroheptyl) thiobenzoate, S-(3,3,7,7,7-pentafluoroheptyl) thiobenzoate, S-(3,4,4,5,5-pentafluoroheptyl) thiobenzoate, S-(3,4,4,5,6-pentafluoroheptyl) thiobenzoate, S-(3,4,4,5,7-pentafluoroheptyl) thiobenzoate, S-(3,4,4,6,7-pentafluoroheptyl) thiobenzoate, S-(3,4,4,7,7-pentafluoroheptyl) thiobenzoate, S-(3,4,5,5,6-pentafluoroheptyl) thiobenzoate, S-(3,4,5,5,7-pentafluoroheptyl) thiobenzoate, S-(3,4,5,6,6-pentafluoroheptyl) thiobenzoate, S-(3,4,5,6,7-pentafluoroheptyl) thiobenzoate, S-(3,4,5,7,7-pentafluoroheptyl) thiobenzoate, S-(3,4,6,6,7-pentafluoroheptyl) thiobenzoate, S-(3,4,6,7,7-pentafluoroheptyl) thiobenzoate, S-(3,4,7,7,7-pentafluoroheptyl) thiobenzoate, S-(3,5,5,6,6-pentafluoroheptyl) thiobenzoate, S-(3,5,5,6,7-pentafluoroheptyl) thiobenzoate, S-(3,5,5,7,7-pentafluoroheptyl) thiobenzoate, S-(3,5,6,6,7-pentafluoroheptyl) thiobenzoate, S-(3,5,6,7,7-pentafluoroheptyl) thiobenzoate, S-(3,5,7,7,7-pentafluoroheptyl) thiobenzoate, S-(3,6,6,7,7-pentafluoroheptyl) thiobenzoate, S-(3,6,7,7,7-pentafluoroheptyl) thiobenzoate, S-(4,4,5,5,6-pentafluoroheptyl) thiobenzoate, S-(4,4,5,5,7-pentafluoroheptyl) thiobenzoate, S-(4,4,5,6,6-pentafluoroheptyl) thiobenzoate, S-(4,4,5,6,7-pentafluoroheptyl) thiobenzoate, S-(4,4,5,7,7-pentafluoroheptyl) thiobenzoate, S-(4,4,6,6,7-pentafluoroheptyl) thiobenzoate, S-(4,4,6,7,7-pentafluoroheptyl) thiobenzoate, S-(4,4,7,7,7-pentafluoroheptyl) thiobenzoate, S-(4,5,5,6,6-pentafluoroheptyl) thiobenzoate, S-(4,5,5,6,7-pentafluoroheptyl) thiobenzoate, S-(4,5,5,7,7-pentafluoroheptyl) thiobenzoate, S-(4,5,7,7,7-pentafluoroheptyl) thiobenzoate, S-(4,6,6,7,7-pentafluoroheptyl) thiobenzoate, S-(4,6,7,7,7-pentafluoroheptyl) thiobenzoate, S-(5,5,6,6,6-pentafluoroheptyl) thiobenzoate, S-(5,5,6,6,7-pentafluoroheptyl) thiobenzoate, S-(5,5,6,7,7-pentafluoroheptyl) thiobenzoate, S-(5,5,7,7,7-pentafluoroheptyl) thiobenzoate, S-(5,6,6,7,7-pentafluoroheptyl) thiobenzoate, S-(5,6,7,7,7-pentafluoroheptyl) thiobenzoate, S-(6,6,7,7,7-pentafluoroheptyl) thiobenzoate,

S-(3,3,4,4,5,5-hexafluoroheptyl) thiobenzoate, S-(3,3,4,4,5,6-hexafluoroheptyl) thiobenzoate, S-(3,3,4,4,5,7-hexafluoroheptyl) thiobenzoate, S-(3,3,4,4,6,6-hexafluoroheptyl) thiobenzoate, S-(3,3,4,4,6,7-hexafluoroheptyl) thiobenzoate, S-(3,3,4,4,7,7-hexafluoroheptyl) thiobenzoate, S-(3,3,4,5,5,6-hexafluoroheptyl) thiobenzoate, S-(3,3,4,5,5,7-hexafluoroheptyl) thiobenzoate, S-(3,3,4,5,6,6-hexafluoroheptyl) thiobenzoate, S-(3,3,4,5,6,7-hexafluoroheptyl) thiobenzoate, S-(3,3,4,5,7,7-hexafluoroheptyl) thiobenzoate, S-(3,3,4,6,6,7-hexafluoroheptyl) thiobenzoate, S-(3,3,4,6,7,7-hexafluoroheptyl) thiobenzoate, S-(3,3,4,7,7,7-hexafluoroheptyl) thiobenzoate, S-(3,3,5,5,6,6-hexafluoroheptyl) thiobenzoate, S-(3,3,5,5,6,7-hexafluoroheptyl) thiobenzoate, S-(3,3,5,5,7,7-hexafluoroheptyl) thiobenzoate, S-(3,3,5,6,6,7-hexafluoroheptyl) thiobenzoate, S-(3,3,5,6,7,7-hexafluoroheptyl) thiobenzoate, S-(3,3,5,7,7,7-hexafluoroheptyl) thiobenzoate, S-(3,3,6,6,7,7-hexafluoroheptyl) thiobenzoate, S-(3,3,6,7,7,7-hexafluoroheptyl) thiobenzoate, S-(3,4,4,5,5,6-hexafluoroheptyl) thiobenzoate, S-(3,4,4,5,5,7-hexafluoroheptyl) thiobenzoate, S-(3,4,4,5,6,6-hexafluoroheptyl) thiobenzoate, S-(3,4,4,5,6,7-hexafluoroheptyl) thiobenzoate, S-(3,4,4,5,7,7-hexafluoroheptyl) thiobenzoate, S-(3,4,4,6,6,7-hexafluoroheptyl) thiobenzoate, S-(3,4,4,6,7,7-hexafluoroheptyl) thiobenzoate, S-(3,4,4,7,7,7-hexafluoroheptyl) thiobenzoate, S-(3,4,5,5,6,6-hexafluoroheptyl) thiobenzoate, S-(3,4,5,5,6,7-hexafluoroheptyl) thiobenzoate, S-(3,4,5,5,7,7-hexafluoroheptyl) thiobenzoate, S-(3,4,5,6,6,7-hexafluoroheptyl) thiobenzoate, S-(3,4,5,6,7,7-hexafluoroheptyl) thiobenzoate, S-(3,4,5,7,7,7-hexafluoroheptyl) thiobenzoate, S-(3,4,6,6,7,7-hexafluoroheptyl) thiobenzoate, S-(3,4,6,7,7,7-hexafluoroheptyl) thiobenzoate, S-(3,5,5,6,6,7-hexafluoroheptyl) thiobenzoate, S-(3,5,5,6,7,7-hexafluoroheptyl) thiobenzoate, S-(3,5,5,7,7,7-hexafluoroheptyl) thiobenzoate, S-(3,5,6,6,7,7-hexafluoroheptyl) thiobenzoate, S-(3,5,6,7,7,7-hexafluoroheptyl) thiobenzoate, S-(3,6,6,7,7,7-hexafluoroheptyl) thiobenzoate, S-(4,4,5,5,6,6-hexafluoroheptyl) thiobenzoate, S-(4,4,5,5,6,7-hexafluoroheptyl) thiobenzoate, S-(4,4,5,5,7,7-hexafluoroheptyl) thiobenzoate, S-(4,4,5,6,7,7-hexafluoroheptyl) thiobenzoate, S-(4,4,5,7,7,7-hexafluoroheptyl) thiobenzoate, S-(4,4,6,6,7,7-hexafluoroheptyl) thiobenzoate, S-(4,4,6,7,7,7-hexafluoroheptyl) thiobenzoate, S-(4,5,5,6,6,7-hexafluoroheptyl) thiobenzoate, S-(4,5,5,6,7,7-hexafluoroheptyl) thiobenzoate, S-(4,5,5,7,7,7-hexafluoroheptyl) thiobenzoate, S-(4,5,6,6,7,7-hexafluoroheptyl) thiobenzoate, S-(4,5,6,7,7,7-hexafluoroheptyl) thiobenzoate, S-(4,6,6,7,7,7-hexafluoroheptyl) thiobenzoate, S-(5,5,6,6,7,7-hexafluoroheptyl) thiobenzoate, S-(5,5,6,7,7,7-hexafluoroheptyl) thiobenzoate, S-(5,6,6,7,7,7-hexafluoroheptyl) thiobenzoate,

S-(3,3,4,4,5,5,6-haptafluoroheptyl) thiobenzoate, S-(3,3,4,4,5,5,7-haptafluoroheptyl) thiobenzoate, S-(3,3,4,4,5,6,6-haptafluoroheptyl) thiobenzoate, S-(3,3,4,4,5,6,7-haptafluoroheptyl) thiobenzoate, S-(3,3,4,4,5,7,7-haptafluoroheptyl) thiobenzoate, S-(3,3,4,4,6,6,7-haptafluoroheptyl) thiobenzoate, S-(3,3,4,4,6,7,7-haptafluoroheptyl) thiobenzoate, S-(3,3,4,4,7,7,7-haptafluoroheptyl) thiobenzoate, S-(3,3,4,5,5,6,6-haptafluoroheptyl) thiobenzoate, S-(3,3,4,5,5,6,7-haptafluoroheptyl) thiobenzoate, S-(3,3,4,5,5,7,7-haptafluoroheptyl) thiobenzoate, S-(3,3,4,5,6,6,7-haptafluoroheptyl) thiobenzoate, S-(3,3,4,5,6,7,7-haptafluoroheptyl) thiobenzoate, S-(3,3,4,5,7,7,7-haptafluoroheptyl) thiobenzoate, S-(3,3,4,6,6,7,7-haptafluoroheptyl) thiobenzoate, S-(3,3,4,6,7,7,7-haptafluoroheptyl) thiobenzoate, S-(3,3,5,5,6,6,7-haptafluoroheptyl) thiobenzoate, S-(3,3,5,5,6,7,7-haptafluoroheptyl) thiobenzoate, S-(3,3,5,5,7,7,7-haptafluoroheptyl) thiobenzoate, S-(3,3,5,6,6,7,7-haptafluoroheptyl) thiobenzoate, S-(3,3,5,6,7,7,7-haptafluoroheptyl) thiobenzoate, S-(3,3,6,6,7,7,7-haptafluoroheptyl) thiobenzoate, S-(3,4,4,5,5,6,6-haptafluoroheptyl) thiobenzoate, S-(3,4,4,5,5,6,7-haptafluoroheptyl) thiobenzoate, S-(3,4,4,5,5,7,7-haptafluoroheptyl) thiobenzoate, S-(3,4,4,5,6,6,7-haptafluoroheptyl) thiobenzoate, S-(3,4,4,5,6,7,7-haptafluoroheptyl) thiobenzoate, S-(3,4,4,5,7,7,7-haptafluoroheptyl) thiobenzoate, S-(3,4,4,6,6,7,7-haptafluoroheptyl) thiobenzoate, S-(3,4,4,6,7,7,7-haptafluoroheptyl) thiobenzoate, S-(3,4,5,5,6,6,7-haptafluoroheptyl) thiobenzoate, S-(3,4,5,5,6,7,7-haptafluoroheptyl) thiobenzoate, S-(3,4,5,5,7,7,7-haptafluoroheptyl) thiobenzoate, S-(3,4,5,6,6,7,7-haptafluoroheptyl) thiobenzoate, S-(3,4,5,6,7,7,7-haptafluoroheptyl) thiobenzoate, S-(3,4,6,6,7,7,7-haptafluoroheptyl) thiobenzoate, S-(3,5,5,6,6,7,7-haptafluoroheptyl) thiobenzoate, 3-(3,5,5,6,7,7,7-haptafluoroheptyl) thiobenzoate, S-(3,5,6,6,7,7,7-haptafluoroheptyl) thiobenzoate, S-(4,4,5,5,6,6,7-haptafluoroheptyl) thiobenzoate, S-(4,4,5,5,6,7,7-haptafluoroheptyl) thiobenzoate, S-(4,4,5,5,7,7,7-haptafluoroheptyl) thiobenzoate, S-(4,4,5,6,6,7,7-haptafluoroheptyl) thiobenzoate, S-(4,4,5,6,7,7,7-haptafluoroheptyl) thiobenzoate, S-(4,4,6,6,7,7,7-haptafluoroheptyl) thiobenzoate, S-(4,5,5,6,6,7,7-haptafluoroheptyl) thiobenzoate, S-(4,5,5,6,7,7,7-haptafluoroheptyl) thiobenzoate, S-(4,5,6,6,7,7,7-haptafluoroheptyl) thiobenzoate, S-(5,5,6,6,7,7,7-haptafluoroheptyl) thiobenzoate,

S-(3,3,4,4,5,5,6,6-octafluoroheptyl) thiobenzoate, S-(3,3,4,4,5,5,6,7-octafluoroheptyl) thiobenzoate, S-(3,3,4,4,5,5,7,7-octafluoroheptyl) thiobenzoate, S-(3,3,4,4,5,6,6,7-octafluoroheptyl) thiobenzoate, S-(3,3,4,4,5,6,7,7-octafluoroheptyl) thiobenzoate, S-(3,3,4,4,5,7,7,7-octafluoroheptyl) thiobenzoate, S-(3,3,4,4,6,6,7,7-octafluoroheptyl) thiobenzoate, S-(3,3,4,4,6,7,7,7-octafluoroheptyl) thiobenzoate, S-(3,3,4,5,5,6,6,7-octafluoroheptyl) thiobenzoate, S-(3,3,4,5,5,6,7,7-octafluoroheptyl) thiobenzoate, S-(3,3,4,5,5,7,7,7-octafluoroheptyl) thiobenzoate, S-(3,3,4,5,6,6,7,7-octafluoroheptyl) thiobenzoate, S-(3,3,4,5,6,7,7,7-octafluoroheptyl) thiobenzoate, S-(3,3,4,6,6,7,7,7-octafluoroheptyl) thiobenzoate, S-(3,3,5,5,6,6,7,7-octafluoroheptyl) thiobenzoate, S-(3,3,5,5,6,7,7,7-octafluoroheptyl) thiobenzoate, S-(3,3,5,6,6,7,7,7-octafluoroheptyl) thiobenzoate, S-(3,4,4,5,5,6,6,7-octafluoroheptyl) thiobenzoate, S-(3,9,4,5,5,6,7,7-octafluoroheptyl) thiobenzoate, S-(3,4,4,5,5,7,7,7-octafluoroheptyl) thiobenzoate, S-(3,4,4,5,6,6,7,7-octafluoroheptyl) thiobenzoate, S-(3,4,4,5,6,7,7,7-octafluoroheptyl) thiobenzoate, S-(3,4,4,6,6,7,7,7-octafluoroheptyl) thiobenzoate, S-(3,9,5,5,6,6,7,7-octafluoroheptyl) thiobenzoate, S-(3,4,5,5,6,7,7,7-octafluoroheptyl) thiobenzoate, S-(3,4,5,6,6,7,7,7-octafluoroheptyl) thiobenzoate, S-(3,5,5,6,6,7,7,7-octafluoroheptyl) thiobenzoate, S-(4,4,5,5,6,6,7,7-octafluoroheptyl) thiobenzoate, S-(4,4,5,5,6,7,7,7-octafluoroheptyl) thiobenzoate, S-(4,4,5,6,6,7,7,7-octafluoroheptyl) thiobenzoate, S-(4,5,5,6,6,7,7,7-octafluoroheptyl) thiobenzoate, S-(3,3,4,4,5,5,6,6,7-nonafluoroheptyl) thiobenzoate, S-(3,3,4,4,5,5,6,7,7-nonafluoroheptyl) thiobenzoate, S-(3,3,4,4,5,5,7,7,7-nonafluoroheptyl) thiobenzoate, S-(3,3,4,4,5,6,6,7,7-nonafluoroheptyl) thiobenzoate, S-(3,3,4,4,5,6,7,7,7-nonafluoroheptyl) thiobenzoate, S-(3,3,4,4,6,6,7,7,7-nonafluoroheptyl) thiobenzoate, S-(3,3,4,5,5,6,6,7,7-nonafluoroheptyl) thiobenzoate, S-(3,3,4,5,5,6,7,7,7-nonafluoroheptyl) thiobenzoate, S-(3,3,4,5,6,6,7,7,7-nonafluoroheptyl) thiobenzoate, S-(3,3,5,5,6,6,7,7,7-nonafluoroheptyl) thiobenzoate, S-(3,4,4,5,5,6,6,7,7-nonafluoroheptyl) thiobenzoate, S-(3,4,4,5,5,6,7,7,7-nonafluoroheptyl) thiobenzoate, S-(3,4,4,5,6,6,7,7,7-nonafluoroheptyl) thiobenzoate, S-(3,4,5,5,6,6,7,7,7-nonafluoroheptyl) thiobenzoate, S-(4,4,5,5,6,6,7,7,7-nonafluoroheptyl) thiobenzoate, S-(3,3,4,4,5,5,6,6,7,7-decafluoroheptyl) thiobenzoate, S-(3,3,4,4,5,5,6,7,7,7-decafluoroheptyl) thiobenzoate, S-(3,3,4,4,5,6,6,7,7,7-decafluoroheptyl) thiobenzoate, S-(3,3,4,5,5,6,6,7,7,7-decafluoroheptyl) thiobenzoate, S-(3,4,4,5,5,6,6,7,7,7-decafluoroheptyl) thiobenzoate, S-(3,3,4,4,5,5,6,6,7,7,7-undecafluoroheptyl) thiobenzoate, and the like.

Among them, S-(3,3,3-trifluoropropyl) thioacetate, S-(3,3,4,4,4-pentafluorobutyl) thioacetate, S-(3,3,4,4,5,5,5-heptafluoropentyl) thioacetate, S-(3,3,3-trifluoropropyl) thiopropionate, S-(3,3,4,4,4-pentafluorobutyl) thiopropionate, S-(3,3,4,4,5,5,5-heptafluoropentyl) thiopropionate, S-(3,3,3-trifluoropropyl) thiobenzoate, S-(3,3,4,4,4-pentafluorobutyl) thiobenzoate and S-(3,3,4,4,5,5,5-heptafluoropentyl) thiobenzoate are preferable, and S-(3,3,3-trifluoromethyl) thioacetate is more preferable.

### EXAMPLES

Hereinafter, the present invention will be described in more detail by way of Examples. The present invention is not limited to these examples.

Here, the content rate of each compound represented by the unit of "% by area", obtained as a result of analyzing the reaction solution using gas chromatography in the following Examples, is a ratio of each peak area of the gas chromatogram of the objective ester (III), byproducts and the unreacted thiocarboxylic acid (I) to the sum of peak areas of the ester (III), byproducts and the thiocarboxylic acid (I), expressed in terms of percentage.

### Example 1

Under a nitrogen atmosphere, 354 g (3.69 mol) of 3,3,3-trifluoropropene was blown into a solution obtained by dissolving 270 g (3.48 mol) of 98% thioacetic acid in 1.89 kg of tert-butyl methyl ether, while stirring, with maintaining the internal temperature around 20°C. To the mixed solution was added 22.6 g (69.5 mmol) of 95%

### 2,2'-azobis(2,4-dimethyl-4-methoxyvaleronitrile), and the mixture was stirred for 72 hours while maintained at 20 to 26°C.

As a result of analyzing the obtained reaction solution using gas chromatography, it was found that 86.1% by area of S-(3,3,3-trifluoropropyl) thioacetate was contained in the components other than the solvent. The reaction solution was distilled under reduced pressure at 50 Torr, to obtain 398 g of 97.8% by area of colorless S-(3,3,3-trifluoropropyl) thioacetate having a weak sulfur odor (yield of 65.0%).

### Example 2

Under a nitrogen atmosphere, 19.0 g (198 mmol) of 3,3,3-trifluoropropene was blown into a solution obtained by dissolving 15.0 g (193 mmol) of 98% thioacetic acid in 60.0 g of ethyl acetate, while stirring, at an internal temperature of 17 to 20°C. To the mixed solution was added 1.25 g (3.86 mmol) of 95% 2,2'-azobis(2,4-dimethyl-4-methoxyvaleronitrile), and the mixture was stirred for 48 hours while maintained at 17 to 25°C, to obtain 94.3 g of a reaction solution containing S-(3,3,3-trifluoropropyl thioacetate. As a result of analyzing the content of the reaction solution using gas chromatography, it was found that 25.5% by weight of S-(3,3,3-trifluoropropyl) thioacetate was contained, and the conversion rate from thioacetic acid to S-(3,3,3-trifluoropropyl) thioacetate was 72.4%.

### Example 3

Under a nitrogen atmosphere, 12.6 g (131 mmol) of 3,3,3-trifluoropropene was blown into a solution obtained by dissolving 10.0 g (129 mmol) of 98% thioacetic acid in 100.0 g of methanol, while stirring, at an internal temperature of 16 to 19°C. To the mixed solution was added 0.84 g (2.57 mmol) of 95% 2,2'-azobis(2,4-dimethyl-4-methoxyvaleronitrile), and the mixture was stirred for 68 hours while maintained at 17 to 25°C, to obtain 114.3 g of a reaction solution containing S-(3,3,3-trifluoropropyl) thioacetate. As a result of analyzing the content of the reaction solution using gas chromatography, it was found that 2.26% by weight of S-(3,3,3-trifluoropropyl) thioacetate was contained, and the conversion rate from thioacetic acid to S-(3,3,3-trifluoropropyl) thioacetate was 11.7%.

### Example 4

Under a nitrogen atmosphere, 5.07 g (52.8 mmol) of 3,3,3-trifluoropropene was blown into a solution obtained by dissolving 4.00 g (51.5 mmol) of 98% thioacetic acid in 20.0 g of ethyl acetate, while stirring, at an internal temperature of 10 to 20°C. To the mixed solution was added 0.84 g (2.57 mmol) of 95% 2,2'-azobis(2,4-dimethyl-4-methoxyvaleronitrile), and the mixture was stirred for 72 hours at 30°C, to obtain 25.8 g of a reaction solution containing S-(3,3,3-trifluoropropyl) thioacetate. As a result of analyzing the content of the reaction solution using gas chromatography, it was found that 25.1% by weight of S-(3,3,3-trifluoropropyl) thioacetate was contained, and the conversion rate from thioacetic acid to S-(3,3,3-trifluoropropyl) thioacetate was 73.1%.

### Example 5

Under a nitrogen atmosphere, 6.31 g (6.57 mmol) of 3,3,3-trifluoropropene was blown into a solution obtained by dissolving 5.00 g (64.4 mmol) of 98% thioacetic acid and 1.05 g (3.23 mmol) of 95% 2,2'-azobis(2,4-dimethyl-4-methoxyvaleronitrile) in 20.0 g of ethyl acetate for 40 minutes, while stirring, with maintaining an internal temperature of 20 to 26°C. After stirring at 19 to 26°C for 48 hours, as a result of analyzing the obtained reaction solution using gas chromatography, it was found that 82.6% by area of S-(3,3,3-trifluoropropyl) thioacetate was contained in the components other than the solvent.

### Example 6

Under a nitrogen atmosphere, a glass pressure-resistant reaction vessel was cooled to -78°C, and 23.2 g (242 mmol) of 3,3,3-trifluoropropene was charged therein. Thereto were added 7.74 g (99.6 mmol) of 98% thioacetic acid and 1.62 g (4.98 mmol) of 95% 2,2'-azobis(2,4-dimethyl-4-methoxyvaleronitrile), and then the reaction vessel was sealed and heated to room temperature. The maximum pressure in the reaction vessel during reaction was 0.61 MPa. The reaction mixture was stirred at room temperature for 20 hours, then ice-cooled, and the excess gas was vented. After opening, as a result of analyzing the content using gas chromatography, 0% by area of thioacetic acid and 79.0% by area of S-(3,3,3-trifluoropropyl) thioacetate were contained.

### Example 7

Under a nitrogen atmosphere, a stainless (SUS-316) pressure-resistant reaction vessel was cooled to -78°C, and 3.71 g (38.6 mmol) of 3,3,3-trifluoropropene was charged therein. Thereto were added 2.00 g (25.7 mmol) of 98% thioacetic acid and 0.33 g (1.03 mmol) of 95% 2,2'-azobis(2,4-dimethyl-4-methoxyvaleronitrile), then the reaction vessel was sealed and heated to room temperature, and the gauge pressure showed 0.3 MPa. The reaction mixture was stirred at room temperature for 22 hours, then ice-cooled, and the excess gas was vented. After opening, as a result of analyzing the content using gas chromatography, 66.5% by area of thioacetic acid and 7.74% by area of S-(3,3,3-trifluoropropyl) thioacetate were contained.

### Example 8

Under a nitrogen atmosphere, a pressure-resistant reaction vessel made of acid-resistant material HASTELLOY C (registered trademark of Haynes International, Inc.) was cooled to -78°C, and 4.83g (50.3 mmol of 3,3,3-trifluoropropene was charged therein. Thereto were added 0.83 g (10.7 mmol) of 98% thioacetic acid and 32.2 mg (0.099 mmol) of 95% 2,2'-azobis(2,4-dimethyl-4-methoxyvaleronitrile), and then the reaction vessel was sealed and heated to room temperature. The reaction mixture was stirred at room temperature for 24 hours, then ice-cooled, and the excess gas was vented. After opening, as a result of analyzing the content using gas chromatography, 88.2% by area of S-(3,3,3-trifluoropropyl) thioacetate was contained.

### Reference Example 1

Under a nitrogen atmosphere, while a mixture obtained by dissolving 6.18 g (64.4 mmol) of 3,3,3-trifluoropropene in 40.0 g of tert-butyl methyl ether was stirred at 26°C under light shielding, 5.00 g (64.4 mmol) of 98% thioacetic acid was added thereto. The mixture was stirred under light shielding for 72 hours with maintained at 21 to 27°C, as a result of analyzing the obtained reaction solution using gas chromatography, 95.7% by area of thioacetic acid and 0.96% by area of S-(3,3,3-trifluoropropyl) thioacetate were contained in the components other than the solvent.

### Reference Example 2

Under a nitrogen atmosphere, 16.4 g (171 mmol) of 3,3,3-trifluoropropene was blown into a solution obtained by dissolving 10.0 g (129 mmol) of 98% thioacetic acid in 70.0 g of ethyl acetate, while stirring, at an internal temperature of 19 to 22°C. The mixture solution was stirred for 8 days while maintained at 20 to 26°C under fluorescent light, to obtain 93.3 g of a reaction solution containing S-(3,3,3-trifluoropropyl) thioacetate. As a result of analyzing the content of the reaction solution using gas chromatography, it was found that 6.6% by weight of S-(3,3,3-trifluoropropyl) thioacetate was contained, and the conversion rate from thioacetic acid to S-(3,3,3-trifluoropropyl) thioacetate was 27.9%.

### INDUSTRIAL APPLICABILITY

It is known that a thiocarboxylic acid S-(fluoroalkyl) ester is a synthetic intermediate for an organic sulfur compound that gives an excellent control effect against harmful arthropods. The present invention is industrially applicable as a method for producing a thiocarboxylic acid S-(fluoroalkyl) ester.

## Claims

1. A method for producing a thiocarboxylic acid S-(fluoroalkyl) ester represented by formula (III) 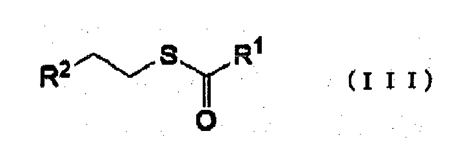 (wherein R¹ represents a methyl group, an ethyl group or a phenyl group, and R² represents a C1-C5 fluoroalkyl group) which comprises reacting a thiocarboxylic acid represented by formula (I) 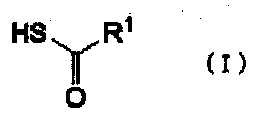 (wherein R¹ has the same meaning as defined above) with a fluoroolefin represented by formula (II) 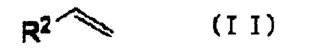 (wherein R² has the same meaning as defined above) in the presence of a radical generator.

2. The production method according to claim 1, wherein the radical generator is an azo compound represented by formula (IV)
**R³-N=N-R⁴** (IV)
(wherein R³ and R⁴ are the same or different, and represent a C2-C12 alkyl group that is optionally substituted or a carbamoyl group).

3. The production method according to claim 1, wherein the radical generator is 2,2'-azobis(4-methoxy-2,4-dimethylvaleronitrile).

4. The production method according to any one of claims 1 to 3, wherein R¹ is a methyl group.

5. The production method according to any one of claims 1 to 4, wherein R² is a C1-C5 perfluoroalkyl group.

6. The production method according to any one of claims 1 to 5, wherein R² is a trifluoromethyl group.

7. The production method according to any one of claims 1 to 6, comprising reacting under atmospheric pressure using an organic solvent.
